# EUROPÄISCHE PATENTSCHRIFT

(11) **EP 3 463 270 B1**
(45) Veröffentlichungstag und Bekanntmachung des Hinweises auf die Patenterteilung: **25.10.2023**
(21) Anmeldenummer: 17726324.1
(22) Anmeldetag: 31.05.2017
(51) Int. Cl.: A61K 8/362, A61K 8/44, A61K 8/49, A61Q 5/06, A61Q 5/08, A61Q 5/10, A61K 8/22, A61K 8/34, A61K 8/365, A61K 8/41

(54) **HAAR SCHONENDE MITTEL UND VERFAHREN ZUR OXIDATIVEN HAARFÄRBUNG ODER BLONDIERUNG MIT AUSGEWÄHLTEN DICARBONSÄUREN**
HAIR-CARE AGENT AND METHOD FOR OXIDATIVE HAIR DYING OR BLEACHING WITH SELECTED DICARBOXYLIC ACIDS
PRODUIT ET PROCÉDÉ DE DÉCOLORATION OU COLORATION D'OXYDATION DOUX POUR LES CHEVEUX FAISANT APPEL À DES ACIDES DICARBOXYLIQUES SÉLECTIONNÉS

(30) Priorität: 31.05.2016 DE 102016209468
(43) Veröffentlichungstag der Anmeldung: 10.04.2019
(73) Patentinhaber: Henkel AG & Co. KGaA, 40589 Düsseldorf (DE)
(72) Erfinder: MANNECK, Hartmut, 23858 Barnitz (DE); HIPPE, Thomas, 25482 Appen (DE); KLEEN-FEHRES, Astrid, 20457 Hamburg (DE)
(86) Internationale Anmeldenummer: PCT/EP2017/063159
(87) Internationale Veröffentlichungsnummer: WO 2017/207629

(56) Entgegenhaltungen:
- WO-A1-2005/115314
- WO-A1-2014/065274
- WO-A1-2017/085117
- DE-A1- 10 051 774
- DE-A1-102015 222 946
- DE-A1-102015 225 137
- US-A- 3 629 330
- US-A1- 2011 247 644
- US-B1- 6 515 114

## Beschreibung

Die vorliegende Erfindung betrifft ein Haar schonendes Mittel zur oxidativen Haarfärbung oder Blondierung sowie ein schonendes Verfahren zur oxidativen Haarfärbung oder Blondierung, bei dem keratinische Fasern vor oxidativen Einflüssen geschützt bzw. oxidative Haarschäden repariert werden.

Beim oxidativen Färben oder Blondieren von Haaren tritt das Problem auf, dass es aufgrund der aggressiven Agentien zu Schäden an der keratinischen Faser kommen kann. Insbesondere die natürliche Hydrophobizität der keratinischen Faser wird reduziert, da die Färbe- bzw. Aufhellmittel das Haar zunächst penetrationsfähig machen müssen, um ihre Wirkung zu entfalten. Die Wasser abweisende Wirkung ist aber einerseits ein natürlicher Schutz des Haares, andererseits sind vom Verbraucher erwünschte Parameter wie Glanz, Geschmeidigkeit, Griff und "Fallen" der Haare eng mit ihr verknüpft.

Um die genannten Nachteile zu überwinden, sind so genannte Vorbehandlungsmittel auf dem Markt, die das Haar vor dem aggressiven Einfluss schützen sollen. Diese beschweren das Haar aber oft oder beeinträchtigen den Erfolg der im Anschluss stattfindenden Aufhellung bzw. Färbung des Haares; insbesondere die Waschechtheit der Färbung kann durch das Vorbehandlungsmittel verschlechtert sein. Auch sind zahlreiche Nachbehandlungsmittel bekannt, mit denen versucht wird, die bei der oxidativen Färbebehandlung verursachten Haarschädigungen zu reparieren. Alle diese Verfahren erfordern aber ein mehrstufiges Anwendungsverfahren, eben entweder eine dem Färben vor- oder nachgelagerte Applikation eines weiteren Haarbehandlungsmittels. Dies wird vom Verbraucher häufig als lästig wahrgenommen, da bereits die oxidative Färbebehandlung selbst mit mehreren Arbeitsschritten und einer Einwirkzeit von bis zu 60 Minuten sehr aufwändig ist.

Der vorliegenden Erfindung lag die Aufgabe zugrunde, ein Mittel und ein Verfahren zur oxidativen Haarfärbung mit einer Haar schützenden Behandlung bereitzustellen, das die genannten Nachteile überwindet, ohne das Farbergebnis der oxidativen Färbebehandlung negativ zu beeinflussen. Dabei sollten insbesondere ein Färbemittel und ein Verfahren bereitgestellt werden, bei dem das Haar nicht beschwert wird und eine möglichst geringe Haarschädigung auftritt. Weiterhin sollte der erzielte Haarschutz möglichst wenig zeitaufwändig sein und möglichst zusammen mit dem Färbeschritt selbst erfolgen.

Der Einsatz von Dicarbonsäuren wie Bernsteinsäure in der Haarpflege ist Stand der Technik. Diese werden in Shampoos und insbesondere in Conditionern breit eingesetzt, um dort Pflegeeffekte zu entfalten. So offenbart die Patentanmeldung WO 2005/115314A1 ein Verfahren zur Restrukturierung keratinischer Fasern, bei dem die Keratinfasern mit Cystin und mit mindestens einer Dicarbonsäure mit 2 bis 10 Kohlenstoffatomen in Kontakt gebracht werden, wobei bevorzugte Dicarbonsäuren ausgewählt sind aus Oxalsäure, Malonsäure, Bernsteinsäure, Glutarsäure, Adipinsäure, Pimelinsäure, Azelainsäure, Maleinsäure, Fumarsäure und Sorbinsäure und Bernsteinsäure besonders bevorzugt ist. Die Patentanmeldung DE 10051774 A1 beschreibt die Verwendung kurzkettiger Carbonsäuren mit einem Molekulargewicht unter 750 g/mol in kosmetischen Mitteln als Wirkstoff zur Restrukturierung keratinischer Fasern. Die Patentanmeldung EP1174112A offenbart Haarbehandlungsmittel, die neben einer organischen Säure als weitere zwingende Bestandteile ein organisches Lösungsmittel, ein kationisches Tensid und einen höheren Alkohol enthalten und zur Reparatur von Poren in Haaren dienen.

DE102015225137A1 offenbart ein oxidatives Behandlungsmittel für keratinische Fasern, insbesondere für Humanhaar, das einen sauren pH/Wert im Bereich von 2,5 - 6,5 aufweist und mindestens eine Dicarbonsäure mit 2 bis 10 Kohlenstoffatomen und/oder mindestens ein Salz dieser Säure(n), mindestens eine Aminosäure, Wasserstoffperoxid und Wasser enthält.

US3629330A offenbart direktziehende Haarfarbstoffe auf der Basis von Alkyl-substituierten Nitrophenylendiaminen mit verbesserten Färbe- und Echtheitseigenschaften, die auch in Oxidationsfärbemitteln eingesetzt werden können.

WO2014065274A1 offenbart Dauerwellmittel, die zur Reparatur der Haarschäden durch ein reduzierend wirkendes Wellmittel die Applikation einer komplexen Wirkstoffmischung aus mindestens sieben verschiedenen Wirkstoffen lehrt, nämlich einer Aminosäure oder einem Derivat hiervon, insbesondere L-Lysinhydrochlorid, L-Threonin, L-Cystein oder L-Arginin, weiterhin einem Pflanzenextrakt, Hyaluronsäure vom Adsorptionstyp, Sojasamenextrakt, flüssiger Hyaluronsäure, fermentiertem Alkohol und Propolis.

Es wurde nun gefunden, dass oxidative Färbe- und Blondiermittel, die neben typischen Bestandteilen, wie Wasser, Ammoniumhydroxid und/oder Monoethanolamin als Alkalisierungsmittel und einer Peroxidverbindung, wie Wasserstoffperoxid, mindestens eine Dicarbonsäure mit 2 bis 10 Kohlenstoffatomen, ausgewählt aus Maleinsäure und Fumarsäure, und mindestens eine Aminosäure der Formel (VI) enthalten, zu deutlich verbessertem Haarschutz bei der oxidativen Haarbehandlung führen, ohne dass die Ergebnisse der oxidativen Färbe- oder Blondierbehandlung beeinträchtigt werden. Es wurde überraschend gefunden, dass durch den Gehalt an mindestens einer ausgewählten Dicarbonsäure mit 2 bis 10 Kohlenstoffatomen in Kombination mit mindestens einer Aminosäure der Formel (VI) das Haar während des Färbens und/oder des Aufhellens vor Schädigungen durch den hohen pH des Mittels und durch das Oxidationsmittel geschützt wird. Dies ließ sich unter anderem daran feststellen, dass beim anschließenden Kämmen weniger Haarbruch auf trat und das Haar weniger an Elastizität einbüßte, wie sich durch Zug-Dehn-Messungen nachweisen ließ, als nach der Applikation nicht erfindungsgemäßer Färbe- und Blondiermittel. Gegenstand der vorliegenden Erfindung ist in einer ersten Ausführungsform ein oxidatives Färbe- oder Blondiermittel für keratinische Fasern, insbesondere für Humanhaar, enthaltend
a) mindestens eine Dicarbonsäure mit 2 bis 10 Kohlenstoffatomen, ausgewählt aus Maleinsäure und Fumarsäure, und/oder mindestens ein Salz dieser Säure(n),
b) mindestens eine Aminosäure der Formel (VI) 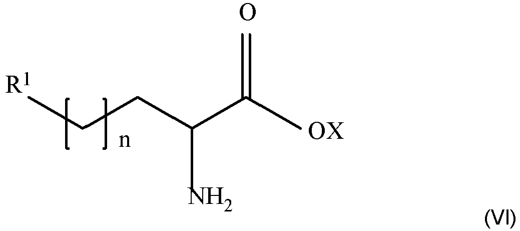 worin
   X für ein Wasserstoffatom oder ein ein- oder zweiwertiges Kation steht;
   n für null, 1, 2 oder 3 steht;
   R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
c) weiterhin mindestens ein Alkalisierungsmittel, ausgewählt aus Ammoniumhydroxid, Monoethanolamin und Natriumsilikaten sowie Mischungen hiervon,
d) ggf. mindestens ein Oxidationsfarbstoffvorprodukt und/oder mindestens einen direktziehenden Farbstoff,
e) Wasser und
f) mindestens eine Peroxidverbindung.

Ein weiterer Gegenstand der vorliegenden Erfindung ist ein Verfahren zur oxidativen Färbung und/ oder Aufhellung keratinischer Fasern, insbesondere von Humanhaar, bei dem ein Färbe- oder Blondiermittel auf die keratinischen Fasern, insbesondere auf das Humanhaar, aufgetragen und nach einer Einwirkzeit von 0,1 bis 60 Minuten, bevorzugt 1 bis 45 Minuten, besonders bevorzugt 10 bis 30 Minuten, wieder ausgespült wird, wobei dieses Färbe- oder Blondiermittel
a) mindestens eine Dicarbonsäure mit 2 bis 10 Kohlenstoffatomen, ausgewählt aus Maleinsäure und Fumarsäure, und/oder mindestens ein Salz dieser Säure(n),
b) mindestens eine Aminosäure der Formel (VI) 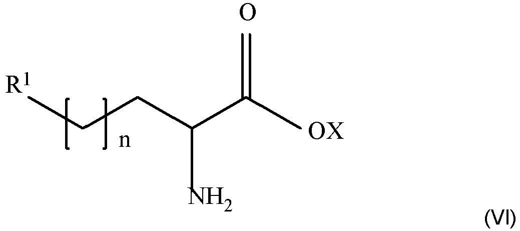 worin
   X für ein Wasserstoffatom oder ein ein- oder zweiwertiges Kation steht;
   n für null, 1, 2 oder 3 steht;
   R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
c) weiterhin mindestens ein Alkalisierungsmittel, ausgewählt aus Ammoniumhydroxid, Monoethanolamin und Natriumsilikaten sowie Mischungen hiervon,
d) ggf. mindestens ein Oxidationsfarbstoffvorprodukt und/oder mindestens einen direktziehenden Farbstoff,
e) Wasser und
f) mindestens eine Peroxidverbindung enthält.

Ein weiterer Gegenstand der vorliegenden Erfindung ist ein Verfahren zur oxidativen Färbung und/oder Aufhellung keratinischer Fasern, insbesondere von Humanhaar, das folgende Verfahrensschritte umfasst:
I. Bereitstellen einer Zusammensetzung (A), enthaltend
   a) mindestens eine Dicarbonsäure mit 2 bis 10 Kohlenstoffatomen, ausgewählt aus Maleinsäure und Fumarsäure, und/oder mindestens ein Salz dieser Säure(n),
   b) mindestens eine Aminosäure der Formel (VI) 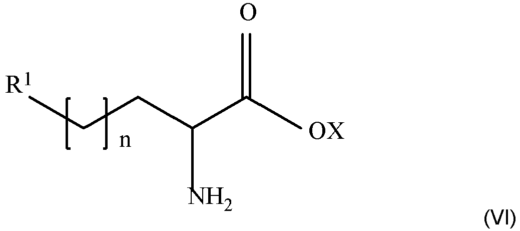 worin
      X für ein Wasserstoffatom oder ein ein- oder zweiwertiges Kation steht;
      n für null, 1, 2 oder 3 steht;
      R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
   c) Wasser und
   d) optional weiterhin mindestens eine Substanz, die ausgewählt ist aus
      - Verbindungen der allgemeinen Formel (III), 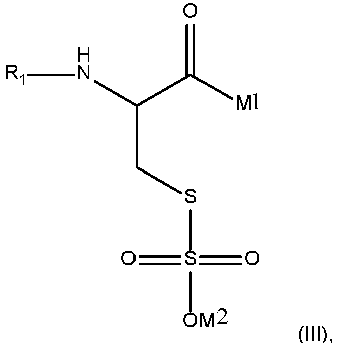 wobei
         R1 für ein Wasserstoffatom oder für ein Strukturelement der Formel (IV) steht 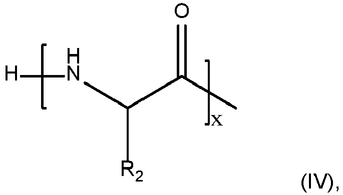 wobei
         x für eine ganze Zahl von 1 bis 100 steht,
         der Rest R2 in jedem der Strukturelemente der Formel (IV) jeweils unabhängig vom vorangegangenen Strukturelement der Formel (IV) gewählt werden kann,
         R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
         M1 steht für die Gruppierung -OM2 oder für ein Strukturelement der Formel (V) 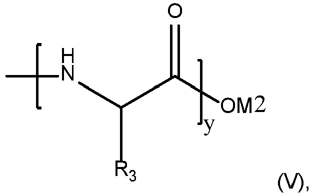 wobei
            y für eine ganze Zahl von 1 bis 100 steht,
            der Rest R3 in jedem der Strukturelemente der Formel (V) jeweils unabhängig vom vorangegangenen Strukturelement der Formel (V) gewählt werden kann,
            R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
            M2 steht für ein Wasserstoffatom, ein Äquivalent eines ein oder mehrwertigen Kations oder ein Ammoniumion (NH₄)⁺, und
      - Polymeren A, die mindestens zehn konstitutive Einheiten der Formel (I) aufweisen, 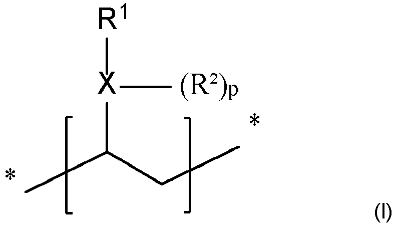 worin
      - X für Stickstoff oder Sauerstoff steht und
      - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
      - p = 0 ist, wenn X für Sauerstoff steht und p = 1 ist, wenn X für Stickstoff steht, wobei das Polymer A keine permanent-ionischen konstitutiven Einheiten enthält,
II. Bereitstellen einer Zusammensetzung (B), enthaltend
   e) mindestens ein Alkalisierungsmittel, ausgewählt aus Ammoniumhydroxid, Monoethanolamin und Natriumsilikaten sowie Mischungen hiervon,
   f) ggf. Wasser und
   g) ggf. mindestens ein Oxidationsfarbstoffvorprodukt und/oder mindestens einen direktziehenden Farbstoff,
III. Bereitstellen einer Zusammensetzung (C), enthaltend
   h) mindestens eine Peroxidverbindung, die bevorzugt Wasserstoffperoxid ist,
IV. Vermischen der Zusammensetzungen (A), (B) und (C) miteinander, direkt anschließend
V. Auftragen der Mischung aus (A), (B) und (C) auf die keratinischen Fasern, insbesondere auf das Humanhaar, und
VI. Ausspülen nach einer Einwirkzeit von 0,1 bis 60 Minuten, bevorzugt 1 bis 45 Minuten, besonders bevorzugt 10 bis 30 Minuten,
VII. ggf. weitere Haarbehandlungen, wie Verformen, Konditionieren und/oder Trocknen.

Ein weiterer Gegenstand der vorliegenden Erfindung ist ein Verfahren zur oxidativen Färbung und/oder Aufhellung keratinischer Fasern, insbesondere von Humanhaar, das folgende Verfahrensschritte umfasst:
I. Bereitstellen einer Zusammensetzung (AB), enthaltend
   a) mindestens eine Dicarbonsäure mit 2 bis 10 Kohlenstoffatomen, ausgewählt aus Maleinsäure und Fumarsäure, und/oder mindestens ein Salz dieser Säure(n),
   b) mindestens eine Aminosäure der Formel (VI), 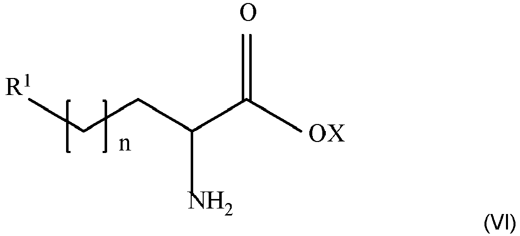 worin
      X für ein Wasserstoffatom oder ein ein- oder zweiwertiges Kation steht;
      n für null, 1, 2 oder 3 steht;
      R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
      wobei die Aminosäure der Formel (VI) bevorzugt ausgewählt ist aus Arginin, Lysin, Histidin sowie Mischungen hiervon, besonders bevorzugt Mischungen aus Arginin und Lysin, oder mindestens einem Salz dieser Aminosäuren,
   c) optional weiterhin mindestens eine Substanz, die ausgewählt ist aus
      - Verbindungen der allgemeinen Formel (III), 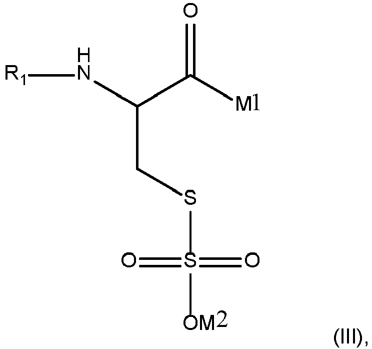 wobei R1 für ein Wasserstoffatom oder für ein Strukturelement der Formel (IV) steht 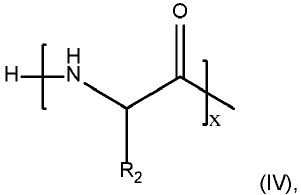 wobei
         x für eine ganze Zahl von 1 bis 100 steht,
         der Rest R2 in jedem der Strukturelemente der Formel (IV) jeweils unabhängig vom vorangegangenen Strukturelement der Formel (IV) gewählt werden kann,
         R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
         M1 steht für die Gruppierung -OM2 oder für ein Strukturelement der Formel (V) 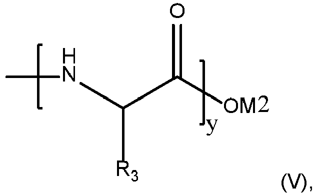 wobei
         y für eine ganze Zahl von 1 bis 100 steht,
         der Rest R3 in jedem der Strukturelemente der Formel (V) jeweils unabhängig vom vorangegangenen Strukturelement der Formel (V) gewählt werden kann,
         R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
         M2 steht für ein Wasserstoffatom, ein Äquivalent eines ein oder mehrwertigen Kations oder ein Ammoniumion (NH₄)⁺, und
      - Polymeren A, die mindestens zehn konstitutive Einheiten der Formel (I) aufweisen, 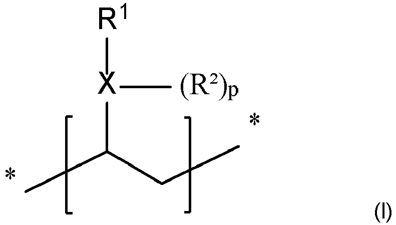 worin
      - X für Stickstoff oder Sauerstoff steht und
      - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
      - p = 0 ist, wenn X für Sauerstoff steht und p = 1 ist, wenn X für Stickstoff steht, wobei das Polymer A keine permanent-ionischen konstitutiven Einheiten enthält,
   d) mindestens ein Alkalisierungsmittel, ausgewählt aus Ammoniumhydroxid, Monoethanolamin und Natriumsilikaten sowie Mischungen hiervon,
   e) Wasser und
   f) ggf. mindestens ein Oxidationsfarbstoffvorprodukt und/oder mindestens einen direktziehenden Farbstoff,
II. Bereitstellen einer Zusammensetzung (C), enthaltend
   g) mindestens eine Peroxidverbindung, die bevorzugt Wasserstoffperoxid ist,
III. Vermischen der Zusammensetzungen (AB) und (C) miteinander, direkt anschließend
IV. Auftragen der Mischung aus (AB) und (C) auf die keratinischen Fasern, insbesondere auf das Humanhaar, und
V. Ausspülen nach einer Einwirkzeit von 0,1 bis 60 Minuten, bevorzugt 1 bis 45 Minuten, besonders bevorzugt 10 bis 30 Minuten,
VI. ggf. weitere Haarbehandlungen, wie Verformen, Konditionieren und/oder Trocknen.

Unter keratinischen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose, verwendet werden.

Dicarbonsäuren mit 2 bis 10 Kohlenstoffatomen und/oder mindestens ein Salz dieser Säure(n) Erfindungsgemäße Dicarbonsäuren mit 2 bis 10 Kohlenstoffatomen sind ausgewählt aus Maleinsäure und Fumarsäure, sowie Mischungen dieser Säuren.

Die genannten Dicarbonsäuren leisten einen wesentlichen Beitrag zur verringerten Haarschädigung durch die erfindungsgemäßen Färbe- oder Blondiermittel.

Je nach pH-Wert des erfindungsgemäßen Färbe- oder Blondiermittels oder der in einem der erfindungsgemäßen Färbe- oder Blondierverfahren eingesetzten Zusammensetzungen (A) oder (AB) kann die mindestens eine Dicarbonsäure mit 2 bis 10 Kohlenstoffatomen, ausgewählt aus Maleinsäure und Fumarsäure, als undissoziierte Säure, teilweise dissoziiert oder vollständig dissoziiert vorliegen. Liegt die mindestens eine Dicarbonsäure mit 2 bis 10 Kohlenstoffatomen teilweise dissoziiert oder vollständig dissoziiert vor, so ist das Gegenion ausgewählt aus physiologisch verträglichen Kationen, wie insbesondere den Alkalimetall-, Erdalkalimetall- und Zinkionen sowie Ammoniumionen, Alkylammonium-, Alkanolammonium- und Glucammoniumionen, insbesondere die Mono-, Di- und Trimethyl-, -ethyl- und -hydroxyethylammoniumionen. Ebenfalls bevorzugt sind die Salze der Dicarbonsäuren mit 2 bis 10 Kohlenstoffatomen, ausgewählt aus Maleinsäure und Fumarsäure, mit Amino-C₁-C₆-Alkanolen, insbesondere mit Monoethanolamin, und Amino-C₁-C₆-Alkandiolen, insbesondere mit 2-Amino-2-methylpropan-1-ol, 2-Amino-2-methylpropan-1,3-diol, 2-Aminopropan-1-ol, 3-Aminopropan-1-ol, 1-Aminopropan-2-ol (MIPA) und 2-Amino-2-(hydroxymethyl)propan-1,3-diol (TRIS), wobei die Salze mit Monoethanolamin, 2-Amino-2-methylpropan-1-ol und 2-Amino-2-methylpropan-1,3-diol besonders bevorzugt sind.

Außerordentlich bevorzugt sind Natrium-, Kalium-, Magnesium-, Ammonium und Monoethanolammonium-lonen als Gegenionen für die teilweise oder vollständig dissoziierten Dicarbonsäuren mit 2 bis 10 Kohlenstoffatomen, ausgewählt aus Maleinsäure und Fumarsäure. Daneben können jedoch auch mit alkalisch reagierenden Aminosäuren, wie beispielsweise Arginin, Lysin, Ornithin und Histidin, neutralisierte Dicarbonsäuren mit 2 bis 10 Kohlenstoffatomen, ausgewählt aus Maleinsäure und Fumarsäure, eingesetzt werden.

Die Natrium-, Kalium-, Ammonium-, Monoethanolammonium-, Lysin- sowie Argininsalze sowie deren Mischungen sind bevorzugte Salze der Dicarbonsäuren mit 2 bis 10 Kohlenstoffatomen, ausgewählt aus Maleinsäure und Fumarsäure.

Bevorzugte erfindungsgemäße Färbe- oder Blondiermittel enthalten die mindestens eine Dicarbonsäure mit 2 bis 10 Kohlenstoffatomen, ausgewählt aus Maleinsäure und Fumarsäure oder ein oder mehrere Salze hiervon in einer Gesamtmenge von 0,2 bis 4 Gew.-%, bevorzugt 0,33 bis 3 Gew.-%, besonders bevorzugt 0,5 bis 2 Gew.-%, jeweils umgerechnet auf die undissoziierte Dicarbonsäure und bezogen auf das Gewicht des Färbe- oder Blondiermittels, wobei die Gesamtmenge an Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure 0,2 bis 4 Gew.-%, bevorzugt 0,33 bis 3 Gew.-%, besonders bevorzugt 0,5 bis 2 Gew.-%, beträgt, jeweils umgerechnet auf die undissoziierte Dicarbonsäure und bezogen auf das Gewicht des Färbe- oder Blondiermittels. Auch wenn die Dicarbonsäuren in Salzform vorliegen, beziehen sich die vorstehenden Mengenangaben auf die jeweilige Dicarbonsäure in undissoziierter Form, um die Mengenangabe nicht durch unterschiedliche Molgewichte der Salze zu verfälschen.

### Aminosäure der Formel (VI)

Die verringerte Haarschädigungswirkung der erfindungsgemäßen Färbe- oder Blondiermittel ist wesentlich zurückzuführen auf die vorgenannten Dicarbonsäuren, ausgewählt aus Maleinsäure und Fumarsäure, im Zusammenwirken mit mindestens einer ausgewählten Aminosäure der Formel (VI).

Die erfindungsgemäßen Färbe- oder Blondiermittel enthalten daher als weitere obligatorische Komponente mindestens eine Aminosäure der Formel (VI) 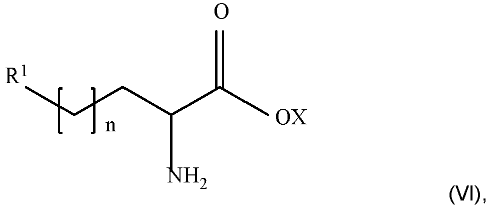 worin
X für ein Wasserstoffatom oder ein ein- oder zweiwertiges Kation steht;
n für null, 1, 2 oder 3 steht;
R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens einem Salz dieser Aminosäure.

Bevorzugte Aminosäuren der Formel (VI) sind ausgewählt aus Arginin, Lysin, Histidin, Asparagin, Glutamin, Cystein, Methionin, Tryptophan sowie Mischungen hiervon. Besonders bevorzugte Färbe- oder Blondiermittel enthalten Mischungen aus Arginin und Lysin oder mindestens einem Salz dieser Aminosäuren.

Bevorzugte erfindungsgemäße Färbe- oder Blondiermittel enthalten die mindestens eine Aminosäure der Formel (VI) oder ein oder mehrere Salze hiervon in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Aminosäure und bezogen auf das Gewicht des Färbe- oder Blondiermittels. Weitere besonders bevorzugte erfindungsgemäße Färbe- oder Blondiermittel enthalten Mischungen aus Arginin und Lysin oder mindestens einem Salz dieser Aminosäuren in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Aminosäure und bezogen auf das Gewicht des Färbe- oder Blondiermittels.

### Alkalisierungsmittel

Die erfindungsgemäßen Färbe- oder Blondiermittel enthalten weiterhin mindestens ein Alkalisierungsmittel, ausgewählt aus Ammoniumhydroxid, Monoethanolamin und Natriumsilikaten sowie Mischungen hiervon.

Zur Erzielung der gewünschten haltbaren Färbung oder Aufhellung der keratinischen Fasern muss das erfindungsgemäße Färbe- oder Blondiermittel einen pH-Wert im Bereich von 6,5 bis 11,0, bevorzugt 8 bis 10,5, besonders bevorzugt 8,5 bis 10, jeweils gemessen bei 20°C, aufweisen. Bei diesen pH-Werten öffnet sich die äußere Keratinfaserschicht optimal zur Aufnahme der Oxidationsfarbstoffvorprodukte und entfaltet sich die gewünschte Wirkung der Peroxidverbindung optimal. Bevorzugt wird Ammoniak in Form seiner wässrigen Lösung eingesetzt. Bei entsprechenden wässrigen Ammoniak-Lösungen kann es sich um 10 bis 35 prozentige Lösungen handeln (berechnet in Gew.-%, 100 g wässrige Ammoniaklösung enthalten dementsprechend 10 bis 35 g Ammoniak). Bevorzugt wird Ammoniak in Form einer 20 bis 30 Gew.-%igen Lösung, besonders bevorzugt in Form einer 25 Gew.-%igen Lösung eingesetzt.

In einer besonders bevorzugten Ausführungsform ist das erfindungsgemäße Färbe- oder Blondiermittel dadurch gekennzeichnet, dass es Ammoniumhydroxid in einer Menge von 0,20 bis 2,5 Gew.-%, bevorzugt von 0,5 bis 2,0 Gew.-%, weiter bevorzugt von 1,0 bis 1,5 Gew.-% und besonders bevorzugt von 0,31 bis 0,8 Gew.-% - bezogen auf das Gesamtgewicht des erfindungsgemäßen Färbe- oder Blondiermittels - enthält.

Zusätzlich zu bzw. anstelle von Ammoniumhydroxid enthalten bevorzugte erfindungsgemäße Färbe- oder Blondiermittel Monoethanolamin
Zur Erzielung einer maximalen Geruchsabdeckung und zur Optimierung der Echtheitseigenschaften ist Monoethanolamin in einer Gesamtmenge von 0,2 - 6,5 Gew.-%, bevorzugt von 0,5 - 4,0 Gew.-%, weiter bevorzugt von 0,7 bis 2,5 Gew.-% und besonders bevorzugt von 0,8 bis 1,6 Gew.-% - bezogen auf das Gesamtgewicht des erfindungsgemäßen Färbe- oder Blondiermittels - enthalten.

Bei Natriumsilikaten im Sinne der vorliegenden Erfindung handelt es sich um chemische Verbindungen, die sich aus Natriumoxid und Siliciumdioxid zusammengesetzen und die in verschiedenen molaren Verhältnissen (Monosilikat, Metasilikat und Polysilikat) vorkommen können. Ein Beispiel für ein Natriumsilikat ist das Natriumsalz der Orthokieselsäure mit der Summenformel Na₄SiO₄, das auch als Natriumorthosilikat bezeichnet wird.

Weitere Beispiele für geeignete Natriumsilikate sind das Dinatriummetasilikat bzw. Natriummetasilikat mit der Summenformel Na₂SiO₃, das Dinatriumdisilikat mit der Summenformel Na₂Si₂O₅ oder das Dinatriumtrisilikat mit der Summenformel Na₂Si₃O₇.

Silikate in amorpher Form können durch das Zusammenschmelzen von Siliciumdioxid und Alkalioxid in molaren Verhältnissen zwischen 1:1 und 4:1 hergestellt werden. Die so erhaltenen Feststoffe werden bei etwa 150 °C und 5 bar Dampfdruck gelöst, um eine Lösung der Natriumsilikate in Wasser zu erhalten, bei diesen entsprechenden Lösungen handelt es sich um Alkaliwassergläser. Als Alkaliwassergläser werden aus einer Schmelze erstarrte, glasartige (amorphe) Natriumsilikate oder ihre wässrigen Lösungen bezeichnet. Diese nennt man auch Natronwasserglas. Auch Natronwassergläser sind innerhalb dieser Erfindung von der Definition der Natriumsilikate umfasst.

Die molare Zusammensetzung bei Wassergläsern beträgt üblicherweise 2 bis 4 mol SiOz auf 1 mol Alkalioxid (NazO).

Ein Beispiel für ein bevorzugtes Natriumsilikat ist Natronwasserglas, das in Form seiner wässrigen Lösung vorliegt, einen NazO-Gehalt von 7,5 bis 8,8 Gew.-% und einen SiO2 Gehalt von 25,0 bis 28,5 Gew.-% besitzt und das die CAS-Nr. 1344-09-5 (Chemical Abstracts Number) hat.

Weitere erfindungsgemäß bevorzugte Färbe- oder Blondiermittel enthalten mindestens ein Natriumsilikat in einer Gesamtmenge von 0,1 bis 9 Gew.-%, bevorzugt 0,2 bis 8 Gew.-%, besonders bevorzugt 1 bis 7,5 Gew.-%, jeweils bezogen auf das Gesamtgewicht des erfindungsgemäßen Färbe- oder Blondiermittels.

Weiterhin können andere Alkalisierungsmittel, wie Kaliumhydroxid (KOH) und Natriumhydroxid (NaOH) enthalten sein, meist in einer Gesamtmenge von 0,05 bis 1,5 Gew.-%, bevorzugt 0,1 bis 0,6 Gew.-%, jeweils bezogen auf das Gesamtgewicht des erfindungsgemäßen Färbe- oder Blondiermittels.

Weiterhin wurde überraschend festgestellt, dass die verringerte Haarschädigungswirkung der erfindungsgemäßen und erfindungsgemäß bevorzugten Färbe- oder Blondiermittel weiter unterstützt werden kann, wenn mindestens eine Verbindung der allgemeinen Formel (III) enthalten ist. Erfindungsgemäß bevorzugte Färbe- oder Blondiermittel enthalten daher
(a) mindestens eine Verbindung der allgemeinen Formel (III) 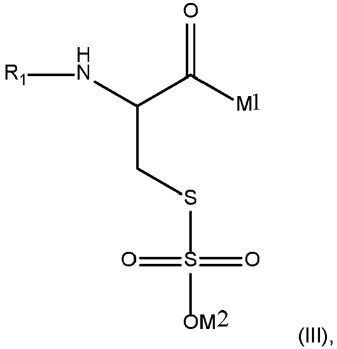 wobei
   R1 für ein Wasserstoffatom oder für ein Strukturelement der Formel (IV) steht 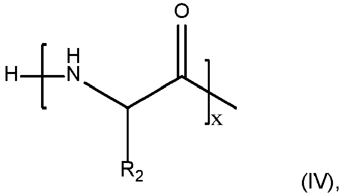
   wobei
   x für eine ganze Zahl von 1 bis 100 steht,
   der Rest R2 in jedem der Strukturelemente der Formel (IV) jeweils unabhängig vom vorangegangenen Strukturelement der Formel (IV) gewählt werden kann,
   R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-yl-methyl-Gruppe oder eine (Sulfosulfanyl)methyl-Gruppe,
   M1 steht für die Gruppierung -OM2 oder für ein Strukturelement der Formel (V) 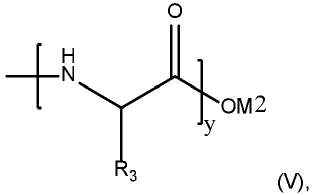
   wobei
   y für eine ganze Zahl von 1 bis 100 steht,
   der Rest R3 in jedem der Strukturelemente der Formel (V) jeweils unabhängig vom vorangegangenen Strukturelement der Formel (V) gewählt werden kann,
   R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M2 steht für ein Wasserstoffatom, ein Äquivalent eines ein oder mehrwertigen Kations oder
   ein Ammoniumion (NH₄)⁺.

Bei dem wesentlichen Inhaltsstoff (a) der Formel (III) handelt es sich um das Bunte-Salz einer Aminosäure, eines Oligopeptids oder eines Peptids, das eine Verbindung der Formel (III) darstellt, 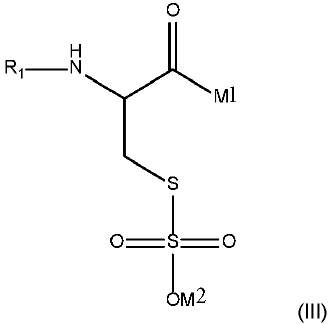 wobei
R1 für ein Wasserstoffatom oder für ein Strukturelement der Formel (IV) steht 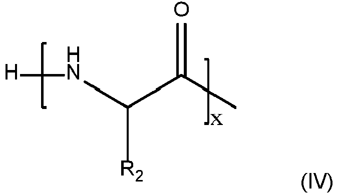
wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-yl-methyl-Gruppe oder eine (Sulfosulfanyl)methyl-Gruppe,
M1 steht für die Gruppierung -OM2 oder für ein Strukturelement der Formel (V) 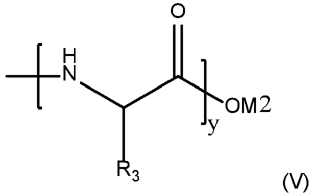
wobei
y für eine ganze Zahl von 1 bis 100 steht
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder
ein Ammoniumion (NH₄)⁺.

Der Rest R1 kann entweder für ein Wasserstoffatom oder für ein Strukturelement der Formel (IV) stehen

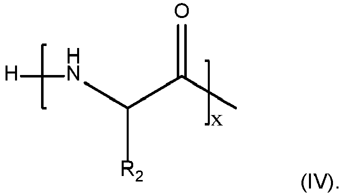

Das Strukturelement der Formel (IV) ist weiterhin gekennzeichnet durch den Wiederholungsindex x, wobei x für eine ganze Zahl von 1 bis 100 steht. Der Wiederholungsindex x gibt an, wie viele Strukturelemente der Formel (IV) in der Verbindung der Formel (III) enthalten sind.

Bevorzugt steht x für eine ganze Zahl von 1 bis 50, weiter bevorzugt steht x für eine ganze Zahl von 1 bis 20, und ganz besonders bevorzugt steht x für eine ganze Zahl von 1 bis 10.

Wenn x beispielweise für die Zahl 10 steht, beinhaltet die Verbindung der Formel (III) 10 Strukturelemente der Formel (IV).

Hierbei ist wesentlich, dass der Rest R2 in jedem der Strukturelemente der Formel (IV) jeweils unabhängig vom vorangegangenen Strukturelement der Formel (IV) gewählt werden kann. Enthalten die Verbindungen der Formel (III) beispielsweise 10 Struktureinheiten der Formel (IV), so können diese 10 Struktureinheiten gleich oder aber auch verschieden sein.

Der Rest R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder für eine (Sulfosulfanyl)methyl-Gruppe.

Bei dem Strukturelement der Formel (IV) handelt es sich somit um eine Aminosäure, die peptidisch über ihre Amino- und/oder ihre Säurefunktion innerhalb der Verbindung der Formel (III) verknüpft ist. Wenn es sich bei der Aminosäure um Cystein handelt, kann diese auch in Form eines Bunte-Salzes vorliegen.

Wenn der Rest R2 für ein Wasserstoffatom steht, so beruht das Strukturelement der Formel (IV) auf der Aminosäure Glycin.

Wenn der Rest R2 für eine Methylgruppe steht, so beruht das Strukturelement der Formel (IV) auf der Aminosäure Alanin.

Wenn der Rest R2 für eine Isopropylgruppe (d.h. eine Gruppe (H₃C)₂CH-) steht, so beruht das Strukturelement der Formel (IV) auf der Aminosäure Valin.

Wenn der Rest R2 für eine 2-Methylpropylgruppe (d.h. eine Gruppe (H₃C)₂CH-CH₂-), so beruht das Strukturelement der Formel (IV) auf der Aminosäure Leucin.

Wenn der Rest R2 für eine 1-Methyl-propylgruppe (d.h. eine Gruppe H3C-CH2-CH(CH3)-) steht, so beruht das Strukturelement der Formel (IV) auf der Aminosäure Isoleucin.

Wenn der Rest R2 für eine Benzylgruppe (d.h. eine Gruppe C₆H₅-CH₂-) steht, so beruht das Strukturelement der Formel (IV) auf der Aminosäure Phenylalanin.

Wenn der Rest R2 für eine 4-Hydroxybenzylgruppe (d.h. eine Gruppe 4-OH-C₆H₅-CH₂-) steht, so beruht das Strukturelement der Formel (IV) auf der Aminosäure Tyrosin.

Wenn der Rest R2 für eine Hydroxymethylgruppe (d.h. eine Gruppe HO-CH2-) steht, so beruht das Strukturelement der Formel (IV) auf der Aminosäure Serin.

Wenn der Rest R2 für eine 1-Hydroxyethylgruppe (d.h. eine Gruppe H3C-CH(OH)-) steht, so beruht das Strukturelement der Formel (IV) auf der Aminosäure Threonin.

Wenn der Rest R2 für eine 4-Aminobutylgruppe (d.h. eine Gruppe H2N-CH2-CH2-CH2-CH2-) steht, so beruht das Strukturelement der Formel (IV) auf der Aminosäure Lysin.

Wenn der Rest R2 für eine 3-Carbamimidamidopropyl-Gruppe (d.h. eine Gruppe H₂N-C(NH)-NH-CH₂-CH₂-CH₂-) steht, so beruht das Strukturelement der Formel (IV) auf der Aminosäure Arginin. Wenn der Rest R2 für eine 2-Carboxyethyl-Gruppe (d.h. eine Gruppe HOOC-CH2-CH2-) steht, so beruht das Strukturelement der Formel (IV) auf der Aminosäure Glutaminsäure.

Wenn der Rest R2 für eine Carboxymethyl-Gruppe (d.h. eine Gruppe HOOC-CH2-) steht, so beruht das Strukturelement der Formel (IV) auf der Aminosäure Asparaginsäure.

Wenn der Rest R2 für eine 2-Carbamoylethyl-Gruppe (d.h. eine Gruppe H2N-C(O)-CH2-CH2-) steht, so beruht das Strukturelement der Formel (IV) auf der Aminosäure Glutamin.

Wenn der Rest R2 für eine Carbamoylmethyl-Gruppe (d.h. eine Gruppe H2N-C(O)-CH2-) steht, so beruht das Strukturelement der Formel (IV) auf der Aminosäure Asparagin.

Wenn der Rest R2 für eine Sulfanylmethyl-Gruppe (d.h. eine Gruppe HS-CH2-) steht, so beruht das Strukturelement der Formel (IV) auf der Aminosäure Cystein.

Wenn der Rest R2 für eine 2-(Methylsulfanyl)ethyl-Gruppe (d.h. eine Gruppe H3C-S-CH2-CH2-) steht, so beruht das Strukturelement der Formel (IV) auf der Aminosäure Methionin.

Wenn der Rest R2 für eine 1H-Imidazol-4-ylmethyl-Gruppe steht, so beruht das Strukturelement der Formel (IV) auf der Aminosäure Histidin.

Wenn der Rest R2 für eine 1H-Indol-3-ylmethyl-Gruppe steht, so beruht das Strukturelement der Formel (IV) auf der Aminosäure Tryptophan.

Schließlich kann der Rest R2 auch für eine (Sulfosulfanyl)methyl-Gruppe stehen, hierbei handelt es sich um eine Bunte-Salz-Struktur der Formel HO-S(Oz)-S-CHz-.

Je nach pH-Wert des Färbe- oder Blondiermittels kann die Bunte-Salz-Struktur der Formel HO-S(Oz)-S-CHz- auch in ihrer deprotonierten Form vorliegen.

Innerhalb der Verbindung der Formel (III) steht M1 steht die Gruppierung -OM2 oder für ein Strukturelement der Formel (V)

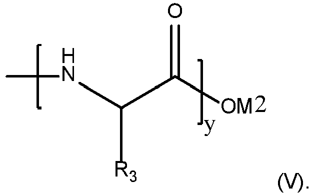

Das Strukturelement der Formel (V) ist gekennzeichnet durch den Wiederholungsindex y, wobei y für eine ganze Zahl von 1 bis 100 steht. Der Wiederholungsindex y gibt an, wie viele Strukturelemente der Formel (V) in der Verbindung der Formel (III) enthalten sind.

Bevorzugt steht y für eine ganze Zahl von 1 bis 50, weiter bevorzugt steht y für eine ganze Zahl von 1 bis 20, und ganz besonders bevorzugt steht y für eine ganze Zahl von 1 bis 10.

Wenn y beispielweise für die Zahl 10 steht, beinhaltet die Verbindung der Formel (III) 10 Strukturelemente der Formel (V).

Hierbei ist wesentlich, dass der Rest R3 in jedem der Strukturelemente der Formel (V) jeweils unabhängig vom vorangegangenen Strukturelement der Formel (V) gewählt werden kann. Enthalten die Verbindungen der Formel (III) beispielsweise 10 Struktureinheiten der Formel (V), so können diese 10 Struktureinheiten gleich oder aber auch verschieden sein.

Der Rest R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe.

Damit handelt es sich auch bei dem Strukturelement der Formel (V) um eine Aminosäure, die peptidisch über ihre Amino- und/oder ihre Säurefunktion innerhalb der Verbindung der Formel (III) verknüpft ist. Wenn es sich bei der Aminosäure um Cystein handelt, kann diese auch in Form eines Bunte-Salzes vorliegen.

Wenn der Rest R3 für ein Wasserstoffatom steht, so beruht das Strukturelement der Formel (V) auf der Aminosäure Glycin.

Wenn der Rest R3 für eine Methylgruppe steht, so beruht das Strukturelement der Formel (V) auf der Aminosäure Alanin.

Wenn der Rest R3 für eine Isopropylgruppe (d.h. eine Gruppe (H₃C)₂CH-) steht, so beruht das Strukturelement der Formel (V) auf der Aminosäure Valin.

Wenn der Rest R3 für eine 2-Methylpropylgruppe (d.h. eine Gruppe (H₃C)₂CH-CH₂-), so beruht das Strukturelement der Formel (V) auf der Aminosäure Leucin.

Wenn der Rest R3 für eine 1-Methyl-propylgruppe (d.h. eine Gruppe H3C-CH2-CH(CH3)-) steht, so beruht das Strukturelement der Formel (V) auf der Aminosäure Isoleucin.

Wenn der Rest R3 für eine Benzylgruppe (d.h. eine Gruppe C₆H₅-CH₂-) steht, so beruht das Strukturelement der Formel (V) auf der Aminosäure Phenylalanin.

Wenn der Rest R3 für eine 4-Hydroxybenzylgruppe (d.h. eine Gruppe 4OH-C₆H₅-CH₂-) steht, so beruht das Strukturelement der Formel (V) auf der Aminosäure Tyrosin.

Wenn der Rest R3 für eine Hydroxymethylgruppe (d.h. eine Gruppe HO-CH2-) steht, so beruht das Strukturelement der Formel (V) auf der Aminosäure Serin.

Wenn der Rest R3 für eine 1-Hydroxyethylgruppe (d.h. eine Gruppe H3C-CH(OH)-) steht, so beruht das Strukturelement der Formel (V) auf der Aminosäure Threonin.

Wenn der Rest R3 für eine 4-Aminobutylgruppe (d.h. eine Gruppe H2N-CH2-CH2-CH2-CH2-) steht, so beruht das Strukturelement der Formel (V) auf der Aminosäure Lysin.

Wenn der Rest R3 für eine 3-Carbamimidamidopropyl-Gruppe (d.h. eine Gruppe H₂N-C(NH)-NH-CH₂-CH₂-CH₂-) steht, so beruht das Strukturelement der Formel (V) auf der Aminosäure Arginin. Wenn der Rest R3 für eine 2-Carboxyethyl-Gruppe (d.h. eine Gruppe HOOC-CH2-CH2-) steht, so beruht das Strukturelement der Formel (V) auf der Aminosäure Glutaminsäure.

Wenn der Rest R3 für eine Carboxymethyl-Gruppe (d.h. eine Gruppe HOOC-CH2-) steht, so beruht das Strukturelement der Formel (V) auf der Aminosäure Asparaginsäure.

Wenn der Rest R3 für eine 2-Carbamoylethyl-Gruppe (d.h. eine Gruppe H2N-C(O)-CH2-CH2-) steht, so beruht das Strukturelement der Formel (V) auf der Aminosäure Glutamin.

Wenn der Rest R3 für eine Carbamoylmethyl-Gruppe (d.h. eine Gruppe H2N-C(O)-CH2-) steht, so beruht das Strukturelement der Formel (V) auf der Aminosäure Asparagin.

Wenn der Rest R3 für eine Sulfanylmethyl-Gruppe (d.h. eine Gruppe HS-CH2-) steht, so beruht das Strukturelement der Formel (V) auf der Aminosäure Cystein.

Wenn der Rest R3 für eine 2-(Methylsulfanyl)ethyl-Gruppe (d.h. eine Gruppe H3C-S-CH2-CH2-) steht, so beruht das Strukturelement der Formel (V) auf der Aminosäure Methionin.

Wenn der Rest R3 für eine 1H-Imidazol-4-ylmethyl-Gruppe steht, so beruht das Strukturelement der Formel (V) auf der Aminosäure Histidin.

Wenn der Rest R3 für eine 1H-Indol-3-ylmethyl-Gruppe steht, so beruht das Strukturelement der Formel (V) auf der Aminosäure Tryptophan.

Schließlich kann der Rest R3 auch für eine (Sulfosulfanyl)methyl-Gruppe stehen, hierbei handelt es sich um eine Bunte-Salz Struktur der Formel HO-S(O₂)-S-CH₂-.

Je nach pH-Wert des Färbe- oder Blondiermittels kann auch hier die Bunte-Salz Struktur der Formel HO-S(O₂)-S-CH₂- auch in ihrer deprotonierten Form vorliegen.

Der Rest M2 steht für ein Wasserstoffatom, ein Äquivalent eines ein oder mehrwertigen Kations oder ein Ammoniumion (NH₄)⁺.

Als bevorzugte Äquivalente eines ein oder mehrwertigen Kations können insbesondere die Kationen von Natrium und Kalium (Na⁺ bzw. K⁺) oder auch Magnesium oder Calcium (1/2 Mg²⁺ oder ½ Ca²⁺) genannt werden.

Steht M2 für ein Wasserstoffatom, so handelt es sich bei der Gruppierung -OM2 um die Gruppierung -OH. Steht M2 für ein Natriumkation, so handelt es sich bei der Gruppierung -OM2 um die Gruppierung -ONa. Steht M2 für ein Kaliumkation, so handelt es sich bei der Gruppierung -OM2 um die Gruppierung -OK. Steht M2 für ein Ammoniumion, so handelt es sich bei der Gruppierung -OM2 um die Gruppierung -O(NH₄).

Die Gruppierung -OM2 ist stets einer Carbonylgruppe benachbart. In Summe liegt, wenn M2 für H, K, Na oder Ammonium steht, damit in der Verbindung der Formel (III) daher entweder in Form eine Säure in ihrer protonierten Form oder aber das Natrium, Kalium oder Ammoniumsalz dieser Säure vor.

Bei den erfindungsgemäßen Verbindungen der Formel (III) handelt es sich entweder um das Bunte-Salz der Aminosäure Cystein, um die Bunte-Salze von Oligo-Peptiden oder aber um die Bunte-Salze von Peptiden.

Wenn der Rest R1 für ein Wasserstoffatom steht und der Rest M1 für eine Gruppierung -OM2 steht, dann handelt es sich bei der Verbindung der Formel (III) um das Bunte-Salz der Aminosäure Cystein. In diesem Fall handelt es sich bei der Verbindung der Formel (III) um die Verbindung der Formel (Illa), 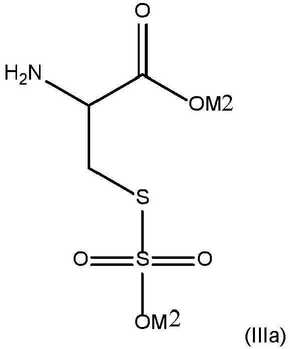 wobei M2 wieder wie zuvor beschrieben definiert.

Liegt die Verbindung der Formel (Illa) in Form ihrer freien Säure vor, so handelt es sich um 2-Amino-3-(sulfosulfanyl)propansäure. Diese Substanz ist kommerziell erhältlich.

Es hat sich herausgestellt, dass der Einsatz der Verbindung der Formel (Illa) in Färbe- oder Blondiermittel bereits bei besonders geringen Einsatzmengen zu einer besonders effektiven Reduzierung der Haarschädigung führt, die auch nach wiederholten Haarwäschen noch vorhanden ist. Deshalb ist der Einsatz von Verbindungen der Formel (Illa) in Färbe- oder Blondiermitteln ganz besonders bevorzugt.

In einer ganz besonders bevorzugten Ausführungsform ist ein erfindungsgemäßes Färbe- oder Blondiermittel, dadurch gekennzeichnet, dass es mindestens eine Verbindung der Formel (III) enthält, wobei
R1 für ein Wasserstoffatom steht und
M1 für eine Gruppierung -OM2 steht.

Wenn eine Verbindung der Formel (Illa) eingesetzt wird, so handelt es sich bevorzugt um den Einsatz dieser spezifischen Verbindung. Werden als Verbindungen der Formel (III) jedoch die Bunte-Salze von Oligopeptiden eingesetzt, so kann das erfindungsgemäße Färbe- oder Blondiermittel auch mehrere Verbindungen der Formel (III) als Gemisch verschiedener Oligopeptide enthalten.

Diese Oligopeptide werden durch ihr mittleres Molgewicht definiert. Das mittlere Molekulargewicht M_{w} des mindestens einen Oligopeptids der Formel (III) kann beispielsweise durch Gelpermeationschromatographie (GPC) mit Polystyrol als internem Standard gemäß DIN 55672-3, Version 8/2007, bestimmt werden.

Je nachdem, wie viele Strukturelemente der Formel (IV) und/oder (V) in der Verbindung der Formel (III) enthalten sind und je nach Art dieser Aminosäuren kann das Molgewicht der erfindungsgemäß verwendeten Verbindung der Formel (III) variieren. Es ist erfindungsgemäß besonders bevorzugt, wenn es sich bei der Verbindung der Formel (III) um ein Oligopeptid handelt, das ein Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Unter dem Begriff "Oligopeptid" im Rahmen der vorliegenden Erfindung werden Kondensationsprodukte von Aminosäuren verstanden, welche die oben genannten Molekulargewichte besitzen.

In einer ganz besonders bevorzugten Ausführungsform ist ein erfindungsgemäßes Färbe- oder Blondiermittel dadurch gekennzeichnet, dass es mindestens eine Verbindung der Formel (III) enthält, die ein Molekulargewicht M_{w} von 200 bis 2.000 Da (Dalton), vorzugsweise von 250 bis 1.500 Da, bevorzugt von 300 bis 1.200 Da, insbesondere von 400 bis 800 Da aufweist.

Wird im erfindungsgemäßen Färbe- oder Blondiermittel eine Mischung von Oligomeren eingesetzt, so können diese Mischungen durch ihr mittleres Molekulargewicht definiert werden.

In diesem Fall ist ein bevorzugtes erfindungsgemäßes Färbe- oder Blondiermittel dadurch gekennzeichnet, dass es mindestens eine Mischung von Verbindungen der Formel (III) enthält, die ein mittleres Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Weiterhin hat sich gezeigt, dass der Schutz- oder Repair-Effekt, den die Verbindungen der Formel (III) besitzen, auch von den Wiederholungsindices x und y abhängt. Wie zuvor beschrieben, ist es ganz besonders bevorzugt, wenn x für eine ganze Zahl von 1 bis 10 steht und y für eine ganze Zahl von 1 bis 10 steht.

In einer weiteren ganz besonders bevorzugten Ausführungsform ist ein erfindungsgemäßes Färbe- oder Blondiermittel dadurch gekennzeichnet, dass es mindestens eine Verbindung der Formel (III) enthält, wobei
R1 für ein Strukturelement der Formel (IV) steht, und
M1 für ein Strukturelement der Formel (V) steht, und
x für eine ganze Zahl von 1 bis 10 steht und
y für eine ganze Zahl von 1 bis 10 steht.

Neben dem Molekulargewicht der Verbindung der Formel (III) nimmt auch der Anteil der Bunte-Salz Einheiten, die in der Verbindung der Formel (III) enthalten sind, einen entscheidenden Einfluss auf die Wirksamkeit der Schutzwirkung oder "Repair-Wirkung" der Verbindungen. Verbindungen mit mindestens einer Bunte-Salz-Einheit - wie sie beispielsweise in der Verbindung der Formel (IIIa) vorhanden ist - sind sehr effektiv, insbesondere wenn sie als monomere Verbindung eingesetzt werden. Oligopeptide mit mindestens einer Bunte-Salz Einheit sind besonders wirksam, wenn sie ein niedriges Molekulargewicht von bis zu 1200, insbesondere 800 Dalton besitzen.

Bei Einsatz von Oligopeptiden ist es jedoch von ganz besonderem Vorteil, wenn die Verbindung der Formel (III) mindestens zwei, bevorzugt mindestens drei Bunte-Salz Einheiten besitzt.

In einer weiteren ganz besonders bevorzugten Ausführungsform ist ein erfindungsgemäßes Färbe- oder Blondiermittel dadurch gekennzeichnet, dass es mindestens eine Verbindung der Formel (III) enthält, wobei
R1 für ein Strukturelement der Formel (IV) steht, und
der Rest R2 in mindestens einem Strukturelement der Formel (IV) für eine
(Sulfosulfanyl)methyl-Gruppe (d.h. eine Gruppe HO-S(O₂)-S-CH₂-) steht.

In einer weiteren ganz besonders bevorzugten Ausführungsform ist ein erfindungsgemäßes Färbe- oder Blondiermittel dadurch gekennzeichnet, dass es mindestens eine Verbindung der Formel (III) enthält, wobei
R1 für ein Strukturelement der Formel (IV) steht, und
x für eine ganze Zahl von mindestens 3 steht und
der Rest R2 in mindestens 3 Strukturelementen der Formel (IV) für eine 2-Carboxyethyl-Gruppe (d.h. eine Gruppe HOOC-CH2-CH2-) steht.

In einer weiteren ganz besonders bevorzugten Ausführungsform ist ein erfindungsgemäßes Färbe- oder Blondiermittel dadurch gekennzeichnet, dass es mindestens eine Verbindung der Formel (III) enthält, wobei
M1 für ein Strukturelement der Formel (V) steht, und
y für eine ganze Zahl von mindestens 3 steht und
der Rest R3 in mindestens 3 Strukturelementen der Formel (IV) für eine Gruppe (Glu) steht. Die mindestens eine Verbindung der Formel (III) ist - bezogen auf das Gesamtgewicht des erfindungsgemäß bevorzugten Färbe- oder Blondiermittels in einer Gesamtmenge von 0,001 bis 10 Gew.-% enthalten. Überraschenderweise hat sich jedoch herausgestellt, dass die Verbindung(en) der Formel (III) bereits in geringen Einsatzkonzentrationen eine sehr gute Reduzierung der Haarschädigung bewirken können. Dies ist insbesondere dann von Vorteil, wenn die mindestens eine Verbindung der Formel (III) dem erfindungsgemäßen Färbe- oder Blondiermittel als Additiv (beispielsweise in Form einer Pflegelösung oder Reparaturlösung) vor der Applikation auf das Haar hinzugefügt werden sollen. Aus diesem Grund ist es besonders vorteilhaft, wenn das erfindungsgemäß bevorzugte Färbe- oder Blondiermittel eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht des erfindungsgemäßen Färbe- oder Blondiermittels.

In einer weiteren ganz besonders bevorzugten Ausführungsform ist ein erfindungsgemäßes Färbe- oder Blondiermittel dadurch gekennzeichnet, dass es eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht des erfindungsgemäßen Färbe- oder Blondiermittels.

### Wasser

Die erfindungsgemäßen Färbe- oder Blondiermittel enthalten Wasser, und zwar bevorzugt in einer Menge von 20 bis 85 Gew.-%, bevorzugt 30 bis 80 Gew.-%, jeweils bezogen auf das Gesamtgewicht des erfindungsgemäßen Färbe- oder Blondiermittels.

### Peroxidverbindungen

Die Ausbildung der Farbstoffe in oxidativen Färbemitteln bzw. der Abbau des haareigenen Farbstoffs Melanin zur Blondierung erfolgt erst durch den Einfluss einer Peroxidverbindung als Oxidationsmittel. Üblicherweise wird hierfür Wasserstoffperoxid verwendet. Der Einsatz von Wasserstoffperoxid kann nur in Form einer wässrigen Lösung erfolgen.

Erfindungsgemäß bevorzugte Färbe- oder Blondiermittel sind dadurch gekennzeichnet, dass sie 0,5 bis 13 Gew.-%, weiter bevorzugt 1 bis 7 Gew.-%, besonders bevorzugt 2 bis 6 Gew.-% und ganz besonders bevorzugt 3 bis 4,5 Gew.-% Wasserstoffperoxid (berechnet als 100 %-iges HzOz) enthalten, jeweils bezogen auf das Gesamtgewicht des erfindungsgemäßen Färbe- oder Blondiermittels.

Blondiermittel oder besonders stark aufhellende Färbemittel können darüber hinaus stark oxidierende Peroxidverbindungen, wie Kalium-, Natrium- und/oder Ammoniumpersulfat, enthalten.

Es hat sich als vorteilhaft erwiesen, wenn die erfindungsgemäßen Oxidationsmittelzubereitungen zur Stabilisierung des Wasserstoffperoxids zusätzlich mindestens einen Stabilisator oder Komplexbildner enthalten. Besonders bevorzugte Stabilisatoren sind insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Sofern es sich bei den erfindungsgemäßen Mitteln um Mittel zum oxidativen Färben von Keratinfasern, insbesondere Humanhaar, handelt, enthalten diese zur Ausbildung der Farbstoffe mindestens ein Oxidationsfarbstoffvorprodukt.

Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind ausgewählt aus der Gruppe 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

In einer bevorzugten Ausführungsform enthalten die erfindungsgemäßen Färbemittel ein oder mehrere Oxidationsfarbstoffvorprodukte in einer Gesamtmenge von 0,01 bis 4,0 Gew.-%, bevorzugt von 0,1 bis 3,5 Gew.-%, weiter bevorzugt von 0,6 bis 3,1 Gew.-% und ganz besonders bevorzugt von 1,2 bis 2,2 Gew.-%, bezogen auf das Gesamtgewicht des erfindungsgemäßen Färbe- oder Blondiermittels.

In einer weiteren bevorzugten Ausführungsform enthalten die erfindungsgemäßen Mittel zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die direktziehenden Farbstoffe sind jeweils bevorzugt in einer Gesamtmenge von 0,001 bis 2 Gew.-%, bezogen auf das Gesamtgewicht des erfindungsgemäßen Färbe- oder Blondiermittels, enthalten. Direktziehende Farbstoffe dienen in oxidativen Färbemitteln zur Nuancierung des erzielten Farbtons, in oxidativen Blondiermitteln zum Ausgleich unerwünschter Rottöne, die beim Abbau des haareigenen Melanins entstehen können. Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethyl-amino-4-nitrophenol.

### Optional: Polymer A mit mindestens 10 konstitutiven Einheiten der Formel (I)

Erfindungsgemäß bevorzugte Oxidationsfärbe- oder -blondiermittel enthalten optional mindestens ein Polymer A, das mindestens zehn konstitutive Einheiten der Formel (I) aufweist, 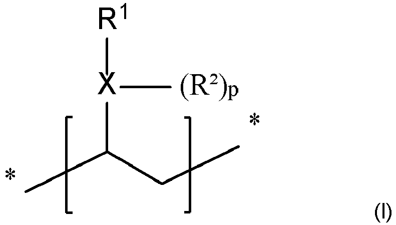 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält.

Überraschend wurde festgestellt, dass ein Polymer A, wie vorstehend bezeichnet und nachstehend näher erläutert, die Schutz- und Reparaturwirkung, die die Kombination aus mindestens einer gesättigten Dicarbonsäure mit 2 bis 10 Kohlenstoffatomen und/oder mindestens einem Salz dieser Säure(n) und mindestens einer Aminosäure der Formel (VI), wie vorstehend erläutert, auf oxidativ gefärbte oder blondierte keratinische Fasern ausübt, in hervorragender Weise unterstützt.

Unter "Polymer" werden im Sinne der vorliegenden Anmeldung Polymere im Sinne der IUPAC-Definition verstanden, die mindestens 10 identische konstitutive Einheiten umfassen.

Gemäß dem RÖMPP Chemie Lexikon, Stand Juli 2009, wird nach einer Definition der IUPAC eine Substanz als Polymer bezeichnet, die sich aus einem Kollektiv chemisch einheitlich aufgebauter Makromoleküle (Polymermoleküle) zusammensetzt, wobei sich diese Makromoleküle oder Polymermoleküle hinsichtlich Polymerisationsgrad, Molmasse und Kettenlänge voneinander unterscheiden. Bei derartigen so genannten polymereinheitlichen Stoffen sind also alle Makromoleküle gleich aufgebaut und unterscheiden sich lediglich durch ihre Kettenlänge (Polymerisationsgrad). Nach dieser IUPAC-Definition ist ein Polymer ferner "ein Polyreaktionsprodukt, das aus einer Vielzahl von Molekülen aufgebaut ist, in denen eine Art oder mehrere Arten von Atomen oder Atomgruppierungen (so genannte konstitutive Einheiten, Grundbausteine oder Wiederholungseinheiten) wiederholt aneinander gereiht sind.

Die Anzahl an konstitutiven Einheiten in einem Polymer bezeichnet man als Polymerisationsgrad. Erfindungsgemäß bevorzugte Polymere A wie auch Polymere B weisen jeweils einen Polymerisationsgrad im Bereich von 40 bis 1000, bevorzugt 100 bis 800, besonders bevorzugt 350 bis 650, auf. Weitere erfindungsgemäß bevorzugte Polymere A mit mindestens zehn konstitutiven Einheit der Formel (I) enthalten 40 bis 1000, bevorzugt 100 bis 800, besonders bevorzugt 350 bis 650 identische konstitutive Einheiten der Formel (I).

R¹ und R² stehen bevorzugt jeweils unabhängig voneinander für Wasserstoff oder für eine C₂-C₁₀-Acylgruppe, die bevorzugt ausgewählt aus einer Acetyl-, Propanoyl-, oder n-Butanoyl-Gruppe, besonders bevorzugt ausgewählt aus einer Acetyl-Gruppe.

Erfindungsgemäß bevorzugte Polymere A weisen mindestens 10 konstitutive Einheiten der Formel (I) auf, in denen X für Stickstoff steht, wobei das Polymer A keine permanent-ionischen konstitutiven Einheiten enthält.

Weitere erfindungsgemäß besonders bevorzugte Polymere A weisen mindestens 10 konstitutive Einheiten der Formel (I) auf, in denen X für Stickstoff steht und R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist.

Wenn R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, so ist dieser Ring bevorzugt mit mindestens einer funktionellen Gruppe substituiert, die ausgewählt ist aus =O. Ein besonders bevorzugte Substituenten-Kombination X, R¹, R² ist eine Pyrrolidon-Gruppe, so dass es sich bei einer erfindungsgemäß besonders bevorzugten konstitutiven Einheit der Formel (I) um eine Einheit der Formel (la) handelt, 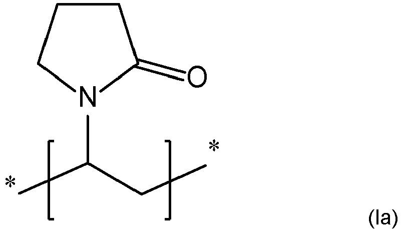 in der X für Stickstoff steht und R¹ und R² zusammen mit diesem Stickstoffatom einen fünfgliedrigen gesättigten Ring bilden, der keine weiteren Heteroatome enthält und der in 2-Position mit einer funktionellen Gruppe =O substituiert ist.

Eine weitere besonders bevorzugte Substituenten-Kombination X, R¹, R² ist eine ε-Caprolactam-Gruppe, so dass es sich bei einer erfindungsgemäß besonders bevorzugten konstitutiven Einheit der Formel (I) um eine Einheit der Formel (I b) handelt, 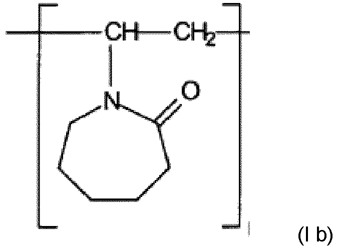 in der X für Stickstoff steht und R¹ und R² zusammen mit diesem Stickstoffatom einen sechsgliedrigen gesättigten Ring bilden, der keine weiteren Heteroatome enthält und der mit einer funktionellen Gruppe =O substituiert ist.

Eine weitere besonders bevorzugte Substituenten-Kombination X, R¹, R² ist eine Imidazol-Gruppe, so dass es sich bei einer weiteren erfindungsgemäß besonders bevorzugten Einheit der Formel (I) um eine Einheit handelt, in der X für Stickstoff steht und R¹ und R² zusammen mit diesem Stickstoffatom einen fünfgliedrigen ungesättigten Ring bilden, der als weiteres Heteroatom Stickstoff enthält.

Weitere erfindungsgemäß bevorzugte Polymere A weisen zu 25 - 100 Mol-%, bevorzugt zu 55 - 100 Mol-%, besonders bevorzugt zu 85 - 100 Mol-% konstitutive Einheiten der Formel (I) auf, in denen X für Stickstoff steht, wobei das Polymer A keine permanent-ionischen konstitutiven Einheiten enthält.

Weitere erfindungsgemäß bevorzugte Polymere A weisen zu 25 - 100 Mol-%, bevorzugt zu 55 - 100 Mol-%, besonders bevorzugt zu 85 - 100 Mol-% konstitutive Einheiten der Formel (I) auf, in denen X für Stickstoff steht und R¹ und R² zusammen mit X einen fünf- oder sechsgliedrigen, gesättigten oder ungesättigten Ring bilden, der gegebenenfalls weitere Heteroatome enthält, die ausgewählt sind aus N und O und gegebenenfalls mit mindestens einer C1 - C6-Alkylgruppe und/oder mit mindestens einer funktionellen Gruppe substituiert ist, wobei das Polymer A keine permanent-ionischen konstitutiven Einheiten enthält.

Erfindungsgemäß besonders bevorzugte Polymere A weisen zu 98 - 100 Mol-% konstitutive Einheiten der Formel (la) auf, wobei das Polymer A keine permanent-ionischen konstitutiven Einheiten enthält.

Erfindungsgemäß außerordentlich bevorzugte Polymere A weisen zu 98 - 100 Mol-% konstitutive Einheiten der Formel (la) auf und haben einen Polymerisationsgrad im Bereich von 40 bis 1000, bevorzugt 100 bis 800, besonders bevorzugt 350 bis 650, wobei das Polymer A keine permanent-ionischen konstitutiven Einheiten enthält. Besonders bevorzugte Polymere A sind Polyvinylpyrrolidon-Homopolymere mit einem Polymerisationsgrad im Bereich von 40 bis 1000, bevorzugt 100 bis 800, besonders bevorzugt 350 bis 650.

Eine weitere besonders bevorzugte Substituenten-Kombination X, R¹, R² ist eine konstitutive Einheit der Formel (I), in der X für Sauerstoff steht, p null ist und R¹ für Wasserstoff steht.

Eine weitere besonders bevorzugte Substituenten-Kombination X, R¹, R² ist eine konstitutive Einheit der Formel (I), in der X für Sauerstoff steht, p null ist und R¹ für eine Acetylgruppe steht. Weitere erfindungsgemäß bevorzugte Polymere A enthalten zu 75-92 Mol-% konstitutive Einheiten der Formel (I), in der X für Sauerstoff steht, p null ist und R¹ für Wasserstoff steht, und zu 8 - 25 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten 40 bis 1000, bevorzugt 100 bis 800, besonders bevorzugt 350 bis 650 konstitutive Einheiten der Formel (I), davon zu 75-92 Mol-% konstitutive Einheiten der Formel (I), in der X für Sauerstoff steht, p null ist und R¹ für Wasserstoff steht, und zu 8 - 25 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten zu 65-25 Mol-% konstitutive Einheiten der Formel (la) und zu 35 - 75 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten 40 bis 1000, bevorzugt 100 bis 800, besonders bevorzugt 350 bis 650 konstitutive Einheiten der Formel (I), davon zu 65-25 Mol-% konstitutive Einheiten der Formel (la) und zu 35 - 75 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Das mindestens eine Polymer A mit mindestens zehn konstitutiven Einheiten der Formel (I) weist keine permanenten ionischen Ladungen auf. Es ist aber möglich, dass die konstitutiven Einheiten der Formel (I), beispielsweise durch Protonierung des Stickstoffatoms in einem sauren Träger, ionisch, insbesondere kationisch, vorliegen. Diese Ladungen sind aber nicht permanent, sondern temporär, da sie vom umgebenden Medium abhängig sind.

Bevorzugte erfindungsgemäße Färbe- oder Blondiermittel enthalten das mindestens eine Polymer A mit mindestens zehn konstitutiven Einheiten der Formel (I) in einer Gesamtmenge von 0,2 bis 5 Gew.-%, bevorzugt 0,5 bis 3 Gew.-%, besonders bevorzugt 1,0 bis 2,3 Gew.-%, jeweils bezogen auf das Gewicht des Färbe- oder Blondiermittels.

Als weiteren optionalen Inhaltsstoff enthalten erfindungsgemäß bevorzugte Färbe- oder Blondiermittel mindestens ein permanent kationisches Polymer B.

Bevorzugt enthält das permanent kationische Polymer neben mindestens einem permanent kationisch geladenen Monomer-Typ auch mindestens einen permanent anionisch geladenen Monomer-Typ, wobei die kationischen Monomere im molaren Überschuss zu den anionischen Monomeren vorliegen, so dass das mindestens eine zweite erfindungsgemäße Polymer eine kationische Nettoladung aufweist. Derartige erfindungsgemäß bevorzugte Polymere werden auch als amphotere oder zwitterionische Polymere bezeichnet.

In einer weiteren bevorzugten Ausführungsform enthalten erfindungsgemäß bevorzugte Färbe- oder Blondiermittel mindestens ein permanent kationisches Polymer, das ausgewählt ist aus - kationischen Polymeren, die aufgebaut sind aus Monomeren mit quartären Ammoniumgruppen der allgemeinen Formel (IIa),

R³-CH=CR⁴-CO-Z-(CₙH₂ₙ)-N⁽⁺⁾R⁵R⁶R⁷ A⁽⁻⁾ (IIa),

in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, bevorzugt ausgewählt aus kationischen Polymeren, die aufgebaut sind aus Acrylamidopropyltrimethylammoniumchlorid, besonders bevorzugt ausgewählt aus amphoteren Polymeren mit kationischer Nettoladung, die durch Polymerisation aufgebaut sind aus
a) kationischen Monomeren mit quartären Ammoniumgruppen der allgemeinen Formel (Ila),

   R³-CH=CR⁴-CO-Z-(CₙH₂ₙ)-N⁽⁺⁾R⁵R⁶R⁷ A⁽⁻⁾ (IIa),

   in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, und
b) mindestens einer ungesättigten Carbonsäure, ausgewählt aus Acrylsäure, Methacrylsäure und Crotonsäure sowie aus Mischungen dieser Säuren, wobei die mindestens eine ungesättigte Carbonsäure in Form ihrer Salze vorliegen kann,

wobei im Polymer die kationischen Monomere im molaren Überschuss zu den anionischen Monomeren vorliegen;
außerordentlich bevorzugt ausgewählt aus amphoteren Polymeren mit kationischer Nettoladung, die den mindestens einen Monomer-Typ der allgemeinen Formel (IIa) und den mindestens einen Monomer-Typ der ungesättigten Carbonsäure, ausgewählt aus Acrylsäure, Methacrylsäure und Crotonsäure sowie Mischungen hiervon, in einem Molverhältnis von 60:40 bis 95:5, bevorzugt von 75:25 bis 90:10 zueinander enthalten,
außerordentlichst bevorzugt ausgewählt aus amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen;
   - 2-[2-Hydroxy-3-(trimethylammonio)propoxy]ethylcelluloseetherchlorid, das beispielsweise unter der INCI-Bezeichnung Polyquaternium-10 erhältlich ist,
   - Terpolymeren aus Acrylsäure, Diallyldimethylammoniumchlorid und Acrylamid, wie sie beispielsweise unter der INCI-Bezeichnung Polyquaternium-39 erhältlich sind,
   - Homopolymeren des N,N,N-Trimethyl-2-[(methyl-1-oxo-2-propenyl)oxy]ethanaminiumchlorid, wie sie beispielsweise unter der INCI-Bezeichnung Polyquaternium-37 erhältlich sind,
   - Copolymeren aus Diallyldimethylammoniumchlorid und Acrylsäure, wie sie beispielsweise unter der INCI-Bezeichnung Polyquaternium-22 erhältlich sind,
   - Hydroxyethylcellulose-Dimethyldiallylammoniumchlorid-Copolymeren, wie sie beispielsweise unter der INCI-Bezeichnung Polyquaternium-4 erhältlich sind,
   - Copolymeren aus Acrylamid und beta-Methacrylyloxyethyltrimethylammoniummethosulfat, wie sie beispielsweise unter der INCI-Bezeichnung Polyquaternium-5 erhältlich sind,
   - Homopolymeren des N,N-Dimethyl-N-2-Propenyl-2-Propen-1-aminiumchlorid, wie sie beispielsweise unter der INCI-Bezeichnung Polyquaternium-6 erhältlich sind,
   - Copolymeren aus Diallyldimethylammoniumchlorid und Acrylamid, wie sie beispielsweise unter der INCI-Bezeichnung Polyquaternium-7 erhältlich sind,
   - Copolymeren aus Vinylpyrrolidon und Dimethylaminoethylmethacrylatdiethylsulfat, wie sie beispielsweise unter der INCI-Bezeichnung Polyquaternium-11 erhältlich sind,
   - sowie Mischungen der vorgenannten Polymere.

Erfindungsgemäß außerordentlich bevorzugte permanente kationische Polymere sind ausgewählt aus 2-[2-Hydroxy-3-(trimethylammonio)propoxy]ethylcelluloseetherchlorid, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen und Terpolymeren aus Acrylsäure, Diallyldimethylammoniumchlorid und Acrylamid, sowie Zweier- und Dreier-Mischungen dieser Polymere.

Besonders bevorzugte Polymer B-Mischungen enthalten 2-[2-Hydroxy-3-(trimethylammonio)propoxy]ethylcelluloseetherchlorid und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten 2-[2-Hydroxy-3-(trimethylammonio)propoxy]ethylcelluloseetherchlorid, mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und mindestens ein Terpolymer aus Acrylsäure, Diallyldimethylammoniumchlorid und Acrylamid.

Erfindungsgemäß ebenfalls außerordentlich bevorzugte permanent-kationische Polymere B sind ausgewählt aus Polyquaternium-10, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen, und Polyquaternium-39, sowie Zweier- und Dreier-Mischungen dieser Polymere.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39.

Bevorzugte erfindungsgemäße Färbe- oder Blondiermittel enthalten das mindestens eine permanent kationische Polymer B in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen Färbe- oder Blondiermittels.

Weitere besonders bevorzugte Färbe- oder Blondiermittel enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen Färbe- oder Blondiermittels.

Weitere besonders bevorzugte Färbe- oder Blondiermittel enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39 in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen Färbe- oder Blondiermittels.

### Optionale Aminosäuren

Optional können die erfindungsgemäßen Färbe- oder Blondiermittel mindestens eine weitere Aminosäure enthalten, die von den Aminosäuren der Formel (VI) verschieden ist. Erfindungsgemäß bevorzugte optionale Aminosäuren sind ausgewählt aus Serin, Alanin, Asparaginsäure, Glutaminsäure, Glycin, Isoleucin, Leucin, Phenylalanin, Prolin, Threonin, Tyrosin und Valin sowie Mischungen dieser Aminosäuren. Die optionalen Aminosäuren können auch in Salzform vorliegen, wobei hier die gleichen Salze bzw. Gegenionen bevorzugt sind wie für die vorstehend genannten Dicarbonsäuren, ausgewählt aus Maleinsäure und Fumarsäure.

Überraschend hat sich herausgestellt, dass ein Gehalt an mindestens einer Aminosäure, die ausgewählt ist aus Serin, Alanin, Asparaginsäure, Glutaminsäure, Glycin, Isoleucin, Leucin, Phenylalanin, Prolin, Threonin, Tyrosin und Valin sowie Mischungen dieser Aminosäuren, die besonders geringe Haarschädigungswirkung der erfindungsgemäßen Färbe- oder Blondiermittel weiter reduzieren kann.

Eine besonders gute Wirkung konnte insbesondere für Serin festgestellt werden. Außerordentlich bevorzugte erfindungsgemäße Färbe- oder Blondiermittel enthalten Serin und mindestens eine der basischen Aminosäuren Arginin, Histidin oder Lysin, wobei besonders bevorzugt Serin und mindestens eine der basischen Aminosäuren Arginin, Histidin oder Lysin im Molverhältnis von Serin zu basischen Aminosäuren insgesamt im Bereich von 1:1 bis 50:1, bevorzugt 5:1 bis 30:1, enthalten sind.

Weitere erfindungsgemäß bevorzugte Färbe- oder Blondiermittel sind dadurch gekennzeichnet, dass die mindestens eine optionale Aminosäure in einer Gesamtmenge von 0,5 bis 5 Gew.-%, bevorzugt 0,7 bis 3 Gew.-%, besonders bevorzugt 0,9 bis 2 Gew.-%, jeweils bezogen auf das Gewicht des Färbe- oder Blondiermittels, enthalten ist.

Ein weiterer Gegenstand der vorliegenden Erfindung ist ein Verfahren zur oxidativen Färbung und/oder Aufhellung keratinischer Fasern, insbesondere von Humanhaar, bei dem ein Färbe- oder Blondiermittel auf die keratinischen Fasern, insbesondere auf das Humanhaar, aufgetragen und nach einer Einwirkzeit von 0,1 bis 60 Minuten, bevorzugt 1 bis 45 Minuten, besonders bevorzugt 10 bis 30 Minuten, wieder ausgespült wird, wobei dieses Färbe- oder Blondiermittel
a) mindestens eine Dicarbonsäure mit 2 bis 10 Kohlenstoffatomen, ausgewählt aus Maleinsäure und Fumarsäure, und/oder mindestens ein Salz dieser Säure(n),
b) mindestens eine Aminosäure der Formel (VI) 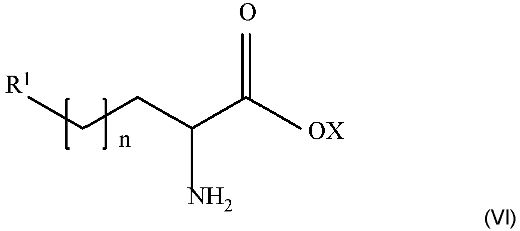 worin
   X für ein Wasserstoffatom oder ein ein- oder zweiwertiges Kation steht;
   n für null, 1, 2 oder 3 steht;
   R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
c) weiterhin mindestens ein Alkalisierungsmittel, ausgewählt aus Ammoniumhydroxid, Monoethanolamin und Natriumsilikaten sowie Mischungen hiervon,
d) ggf. mindestens ein Oxidationsfarbstoffvorprodukt und/oder mindestens einen direktziehenden Farbstoff,
e) Wasser und
f) mindestens eine Peroxidverbindung
enthält.

Für das erfindungsgemäße Verfahren zur oxidativen Färbung und/oder Aufhellung keratinischer Fasern, insbesondere von Humanhaar, und seine bevorzugten Ausführungsformen gilt mutatis mutandis das zu den erfindungsgemäßen und erfindungsgemäß bevorzugten Färbe- und Blondiermitteln Gesagte.

An das erfindungsgemäße Verfahren zur oxidativen Färbung und/oder Aufhellung keratinischer Fasern, insbesondere von Humanhaar, können sich optional weitere Haarbehandlungsschritte anschließen, beispielsweise die Applikation eines Conditioners, eines Haarverformungsmittels, beispielsweise eines Glättmittels oder eines Wellmittels, eines weiteren Haarfärbemittels, beispielsweise zum Färben oder Blondieren von Strähnchen, Ausspülschritte und Trocknungsschritte, zum Beispiel das Trockenreiben oder -pressen mit dem Handtuch, Fönen oder das Trocknen mit einer Trockenhaube.

Es kann erfindungsgemäß bevorzugt sein, die erfindungsgemäße Haar schützende Kombination aus mindestens einer Dicarbonsäure mit 2 bis 10 Kohlenstoffatomen, ausgewählt aus Maleinsäure und Fumarsäure, und/oder mindestens einem Salz dieser Säure(n) und mindestens einer Aminosäure der Formel (VI), wie vorstehend erläutert, zunächst getrennt von der Zubereitung, die das mindestens eine Alkalisierungsmittel und ggf. mindestens ein Oxidationsfarbstoffvorprodukt und/oder mindestens einen direktziehenden Farbstoff enthält, und getrennt von der Oxidationsmittelzubereitung, die mindestens eine Peroxidverbindung enthält, aufzubewahren und erst kurz vor Beginn des erfindungsgemäßen oder erfindungsgemäß bevorzugten Verfahrens zur oxidativen Färbung und/oder Aufhellung der keratinischen Fasern das erfindungsgemäße oder erfindungsgemäß bevorzugte Färbe- oder Blondiermittel durch Vermischen der drei Komponenten herzustellen.

Ein weiterer Gegenstand der vorliegenden Anmeldung ist daher ein Verfahren zur oxidativen Färbung und/oder Aufhellung keratinischer Fasern, insbesondere von Humanhaar, das folgende Verfahrensschritte umfasst:
I. Bereitstellen einer Zusammensetzung (A), enthaltend
   a) mindestens eine Dicarbonsäure mit 2 bis 10 Kohlenstoffatomen, ausgewählt aus Maleinsäure und Fumarsäure, und/oder mindestens ein Salz dieser Säure(n),
   b) mindestens eine Aminosäure der Formel (VI) 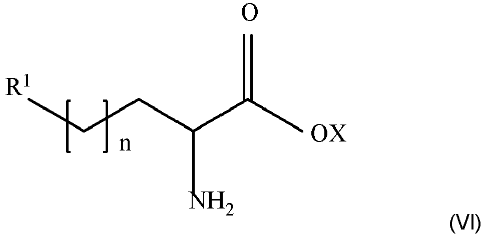 worin
      X für ein Wasserstoffatom oder ein ein- oder zweiwertiges Kation steht;
      n für null, 1, 2 oder 3 steht;
      R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
   c) Wasser und
   d) optional weiterhin mindestens eine Substanz, die ausgewählt ist aus
      - Verbindungen der allgemeinen Formel (III), 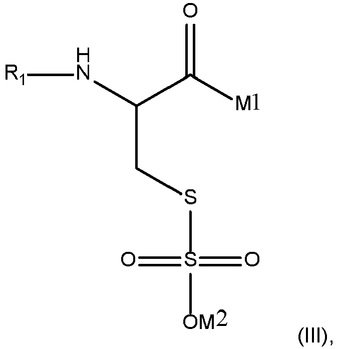 wobei
         R1 für ein Wasserstoffatom oder für ein Strukturelement der Formel (IV) steht 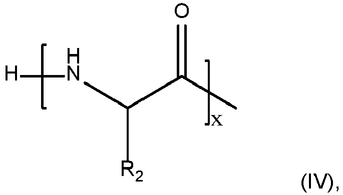
         wobei
         x für eine ganze Zahl von 1 bis 100 steht,
         der Rest R2 in jedem der Strukturelemente der Formel (IV) jeweils unabhängig vom vorangegangenen Strukturelement der Formel (IV) gewählt werden kann,
         R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
         M1 steht für die Gruppierung -OM2 oder für ein Strukturelement der Formel (V) 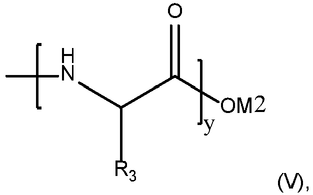
         wobei
         y für eine ganze Zahl von 1 bis 100 steht,
         der Rest R3 in jedem der Strukturelemente der Formel (V) jeweils unabhängig vom vorangegangenen Strukturelement der Formel (V) gewählt werden kann,
         R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
         M2 steht für ein Wasserstoffatom, ein Äquivalent eines ein oder mehrwertigen Kations oder ein Ammoniumion (NH₄)⁺, und
      - Polymeren A, die mindestens zehn konstitutive Einheiten der Formel (I) aufweisen, 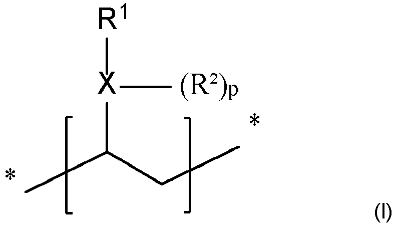 worin
      - X für Stickstoff oder Sauerstoff steht und
      - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
      - p = 0 ist, wenn X für Sauerstoff steht und p = 1 ist, wenn X für Stickstoff steht, wobei das Polymer A keine permanent-ionischen konstitutiven Einheiten enthält,
II. Bereitstellen einer Zusammensetzung (B), enthaltend
   e) mindestens ein Alkalisierungsmittel, ausgewählt aus Ammoniumhydroxid, Monoethanolamin und Natriumsilikaten sowie Mischungen hiervon,
   f) ggf. Wasser und
   g) ggf. mindestens ein Oxidationsfarbstoffvorprodukt und/oder mindestens einen direktziehenden Farbstoff,
III. Bereitstellen einer Zusammensetzung (C), enthaltend
   h) mindestens eine Peroxidverbindung, die bevorzugt Wasserstoffperoxid ist,
IV. Vermischen der Zusammensetzungen (A), (B) und (C) miteinander, direkt anschließend
V. Auftragen der Mischung aus (A), (B) und (C) auf die keratinischen Fasern, insbesondere auf das Humanhaar, und
VI. Ausspülen nach einer Einwirkzeit von 0,1 bis 60 Minuten, bevorzugt 1 bis 45 Minuten, besonders bevorzugt 10 bis 30 Minuten,
VII. ggf. weitere Haarbehandlungen, wie Verformen, Konditionieren und/oder Trocknen.

Mischungsverhältnisse von Zusammensetzung (A), Zusammensetzung (B) und Zusammensetzung (C)

Es hat sich erfindungsgemäß bewährt, wenn das Gewichtsverhältnis aus der Zusammensetzung (A), die Wasser und die Haar schützende Kombination aus mindestens einer Dicarbonsäure mit 2 bis 10 Kohlenstoffatomen, ausgewählt aus Maleinsäure und Fumarsäure, und mindestens einer

Aminosäure der Formel (VI) enthält, der oxidativ färbenden und/oder oxidativ aufhellenden Mischung aus Zusammensetzung (B), enthaltend mindestens ein Alkalisierungsmittel und ggf. Oxidationsfarbstoffvorprodukte und/oder direktziehende Farbstoffe und Zusammensetzung (C), enthaltend mindestens eine Peroxidverbindung, im Bereich von [Gewicht von A]/[[Gewicht von B + Gewicht von C] wie 1:4 bis 1:50, bevorzugt 1:5 bis 1:25, besonders bevorzugt 1:9 bis 1:20, außerordentlich bevorzugt 1:10 bis 1:15.

Erfindungsgemäß bevorzugte Färbe- oder Aufhellverfahren mit den mindestens drei vorgenannten Zusammensetzungen (A), (B) und (C) sind dadurch gekennzeichnet, dass die mindestens eine Dicarbonsäure mit 2 bis 10 Kohlenstoffatomen, ausgewählt aus Maleinsäure und Fumarsäure, in der Zusammensetzung (A) in einer Gesamtmenge von 2 bis 20 Gew.-%, bevorzugt 5 bis 15 Gew.-%, besonders bevorzugt 8 bis 12 Gew.-%, enthalten ist, jeweils umgerechnet auf die undissoziierte Dicarbonsäure und bezogen auf das Gewicht der Zusammensetzung (A), enthalten ist, wobei die Gesamtmenge an Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure bis 20 Gew.-%, bevorzugt 5 bis 15 Gew.-%, besonders bevorzugt 8 bis 12 Gew.-%, beträgt, jeweils umgerechnet auf die undissoziierte Dicarbonsäure und bezogen auf das Gewicht der Zusammensetzung (A). Erfindungsgemäß bevorzugte Färbe- oder Aufhellverfahren mit den mindestens drei vorgenannten Zusammensetzungen (A), (B) und (C) sind weiterhin dadurch gekennzeichnet, dass die mindestens eine Aminosäure der Formel (VI) und/oder ein Salz hiervon in der Zusammensetzung (A) in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), enthalten ist.

Erfindungsgemäß bevorzugte Färbe- oder Aufhellverfahren mit den mindestens drei vorgenannten Zusammensetzungen (A), (B) und (C) sind weiterhin dadurch gekennzeichnet, dass mindestens ein Polymer A, das mindestens zehn konstitutive Einheiten der Formel (I) aufweist, 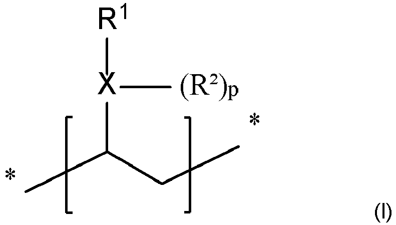 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält,

in der Zusammensetzung (A) in einer Gesamtmenge von 0,5 bis 14 Gew.-%, bevorzugt 1,0 bis 11 Gew.-%, besonders bevorzugt 2,0 bis 10 Gew.-%, jeweils bezogen auf das Gewicht der Zusammensetzung (A), enthalten ist.

Weitere erfindungsgemäß bevorzugte Färbe- oder Aufhellverfahren mit den mindestens drei vorgenannten Zusammensetzungen (A), (B) und (C) sind dadurch gekennzeichnet, dass in Zusammensetzung (A) eine oder mehrere Verbindungen der vorstehend aufgeführten Formel (III) in einer Gesamtmenge von 0,001 bis 1 Gew.-% weiter bevorzugt von 0,005 bis 0,5 Gew.-% und besonders bevorzugt von 0,01 bis 0,1 Gew.-% enthalten ist, jeweils bezogen auf das Gewicht der Zusammensetzung (A).

Weitere erfindungsgemäß bevorzugte Färbe- oder Aufhellverfahren mit den mindestens drei vorgenannten Zusammensetzungen (A), (B) und (C) sind dadurch gekennzeichnet, dass in Zusammensetzung (A) 50 - 92 Gew.-%, bevorzugt 60 - 87 Gew.-% und besonders bevorzugt von 65 bis 80 Gew.-% Wasser enthalten ist, jeweils bezogen auf das Gewicht der Zusammensetzung (A). Weitere erfindungsgemäß bevorzugte Färbe- oder Aufhellverfahren mit den mindestens drei vorgenannten Zusammensetzungen (A), (B) und (C) sind dadurch gekennzeichnet, dass die Zusammensetzung (A) einen pH-Wert im Bereich von 3,5 bis 7,1, bevorzugt 4,5 - 6,5, besonders bevorzugt 5,0 bis 6,0, aufweist, jeweils gemessen bei 20°C.

Weitere erfindungsgemäß bevorzugte Färbe- oder Aufhellverfahren mit den mindestens drei vorgenannten Zusammensetzungen (A), (B) und (C) sind dadurch gekennzeichnet, dass die Zusammensetzung (B) einen pH-Wert im Bereich von 6,5 bis 11,0, bevorzugt 8 - 10,5, besonders bevorzugt 8,5 bis 10,0, aufweist, jeweils gemessen bei 20°C.

Weitere erfindungsgemäß bevorzugte Färbe- oder Aufhellverfahren mit den mindestens drei vorgenannten Zusammensetzungen (A), (B) und (C) sind dadurch gekennzeichnet, dass die Zusammensetzung (C) einen pH-Wert im Bereich von 2,5 bis 6,5, bevorzugt 3,0 - 5,5, besonders bevorzugt 3,5 bis 5,0, aufweist, jeweils gemessen bei 20°C.

Weitere erfindungsgemäß bevorzugte Färbe- oder Aufhellverfahren mit den mindestens drei vorgenannten Zusammensetzungen (A), (B) und (C) sind dadurch gekennzeichnet, dass die Zusammensetzung (C) 1,0 bis 23,0 Gew.-%, weiter bevorzugt 2,5 bis 21,0 Gew.-%, besonders bevorzugt 4,0 bis 20,0 Gew.-% und ganz besonders bevorzugt 5,0 bis 18,0 Gew.-% Wasserstoffperoxid (berechnet als 100 %iges HzOz) enthält, jeweils bezogen auf das Gewicht der Zusammensetzung (C).

Für das erfindungsgemäße Verfahren zur oxidativen Färbung und/oder Aufhellung keratinischer Fasern, insbesondere von Humanhaar, mit dem aus den mindestens drei vorstehend genannten Zusammensetzungen (A), (B) und (C) und seine bevorzugten Ausführungsformen gilt - abgesehen von den geänderten quantitativen Angaben, die vorstehend genannt sind - mutatis mutandis das zu den erfindungsgemäßen und erfindungsgemäß bevorzugten Färbe- und Blondiermitteln Gesagte.

Ein weiterer Gegenstand der vorliegenden Erfindung ist eine Zusammensetzung (A) zur Haarbehandlung, enthaltend
- mindestens eine Dicarbonsäure mit 2 bis 10 Kohlenstoffatomen, in einer Gesamtmenge von 2 bis 20 Gew.-%, bevorzugt 5 bis 15 Gew.-%, besonders bevorzugt 8 bis 12 Gew.-%, jeweils umgerechnet auf die undissoziierte Dicarbonsäure und bezogen auf das Gewicht der Zusammensetzung (A), wobei die Dicarbonsäure ausgewählt ist aus Maleinsäure und Fumarsäure, sowie Mischungen dieser Säuren, wobei die Gesamtmenge an Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure 2 bis 20 Gew.-%, bevorzugt 5 bis 15 Gew.-%, besonders bevorzugt 8 bis 12 Gew.-%, beträgt, jeweils umgerechnet auf die undissoziierte Dicarbonsäure und bezogen auf das Gewicht der Zusammensetzung (A), weiterhin
- mindestens eine Aminosäure der Formel (VI) und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), und
- Wasser, bevorzugt in einer Menge von 50 - 92 Gew.-%, besonders bevorzugt 60 - 87 Gew.-% und außerordentlich bevorzugt von 65 bis 80 Gew.-%, jeweils bezogen auf das Gewicht der Zusammensetzung (A).

Ein weiterer Gegenstand der vorliegenden Erfindung ist eine Zusammensetzung (A) zur Haarbehandlung, enthaltend
- mindestens eine Dicarbonsäure mit 2 bis 10 Kohlenstoffatomen in einer Gesamtmenge von 2 bis 20 Gew.-%, bevorzugt 5 bis 15 Gew.-%, besonders bevorzugt 8 bis 12 Gew.-%, jeweils umgerechnet auf die undissoziierte Dicarbonsäure und bezogen auf das Gewicht der Zusammensetzung (A), wobei die Dicarbonsäure ausgewählt ist aus Maleinsäure und Fumarsäure, sowie Mischungen dieser Säuren, wobei die Gesamtmenge an Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure 2 bis 20 Gew.-%, bevorzugt 5 bis 15 Gew.-%, besonders bevorzugt 8 bis 12 Gew.-%, beträgt, jeweils umgerechnet auf die undissoziierte Dicarbonsäure und bezogen auf das Gewicht der Zusammensetzung (A), weiterhin
- mindestens eine der Aminosäuren Arginin, Histidin oder Lysin und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), und
- 50 - 92 Gew.-%, besonders bevorzugt 60 - 87 Gew.-% und außerordentlich bevorzugt von 65 bis 80 Gew.-% Wasser, jeweils bezogen auf das Gewicht der Zusammensetzung (A).

Ein weiterer Gegenstand der vorliegenden Erfindung ist eine Zusammensetzung (A) zur Haarbehandlung, enthaltend
- mindestens eine Dicarbonsäure mit 2 bis 10 Kohlenstoffatomen in einer Gesamtmenge von 2 bis 20 Gew.-%, bevorzugt 5 bis 15 Gew.-%, besonders bevorzugt 8 bis 12 Gew.-%, jeweils umgerechnet auf die undissoziierte Dicarbonsäure und bezogen auf das Gewicht der Zusammensetzung (A), wobei die Dicarbonsäure ausgewählt ist aus Maleinsäure und Fumarsäure, sowie Mischungen dieser Säuren, wobei die Gesamtmenge an Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure 2 bis 20 Gew.-%, bevorzugt 5 bis 15 Gew.-%, besonders bevorzugt 8 bis 12 Gew.-%, beträgt, jeweils umgerechnet auf die undissoziierte Dicarbonsäure und bezogen auf das Gewicht der Zusammensetzung (A), weiterhin
- mindestens eine Aminosäure der Formel (VI) und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), weiterhin
- Wasser, bevorzugt in einer Menge von 50 - 92 Gew.-%, besonders bevorzugt 60 - 87 Gew.-% und außerordentlich bevorzugt von 65 bis 80 Gew.-%, jeweils bezogen auf das Gewicht der Zusammensetzung (A), und
- mindestens ein Polymer A, das mindestens zehn konstitutive Einheiten der Formel (I) aufweist, 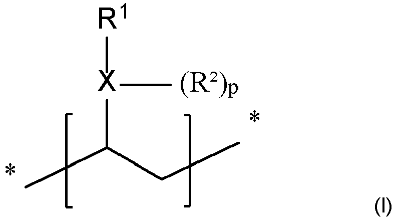 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht, wobei das Polymer A keine permanent-ionischen konstitutiven Einheiten enthält,
in der Zusammensetzung (A) in einer Gesamtmenge von 0,5 bis 14 Gew.-%, bevorzugt 1,0 bis 11 Gew.-%, besonders bevorzugt 2,0 bis 10 Gew.-%, jeweils bezogen auf das Gewicht der Zusammensetzung (A).

Das in erfindungsgemäß bevorzugten Zusammensetzungen (A) enthaltene Polymer A weist mindestens zehn konstitutive Einheiten der Formel (I) auf, 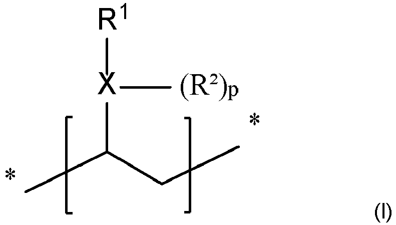 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält.

Überraschend wurde festgestellt, dass ein Polymer A, wie vorstehend bezeichnet und nachstehend näher erläutert, die Schutz- und Reparaturwirkung, die die Kombination aus mindestens einer Dicarbonsäure mit 2 bis 10 Kohlenstoffatomen, ausgewählt aus Maleinsäure und Fumarsäure, und/oder mindestens einem Salz dieser Säure(n) und mindestens einer Aminosäure der Formel (VI), wie vorstehend erläutert, auf oxidativ gefärbte oder blondierte keratinische Fasern ausübt, in hervorragender Weise unterstützt.

Unter "Polymer" werden im Sinne der vorliegenden Anmeldung Polymere im Sinne der IUPAC-Definition verstanden, die mindestens 10 identische konstitutive Einheiten umfassen.

Gemäß dem RÖMPP Chemie Lexikon, Stand Juli 2009, wird nach einer Definition der IUPAC eine Substanz als Polymer bezeichnet, die sich aus einem Kollektiv chemisch einheitlich aufgebauter Makromoleküle (Polymermoleküle) zusammensetzt, wobei sich diese Makromoleküle oder Polymermoleküle hinsichtlich Polymerisationsgrad, Molmasse und Kettenlänge voneinander unterscheiden. Bei derartigen so genannten polymereinheitlichen Stoffen sind also alle Makromoleküle gleich aufgebaut und unterscheiden sich lediglich durch ihre Kettenlänge (Polymerisationsgrad). Nach dieser IUPAC-Definition ist ein Polymer ferner "ein Polyreaktionsprodukt, das aus einer Vielzahl von Molekülen aufgebaut ist, in denen eine Art oder mehrere Arten von Atomen oder Atomgruppierungen (so genannte konstitutive Einheiten, Grundbausteine oder Wiederholungseinheiten) wiederholt aneinander gereiht sind.

Die Anzahl an konstitutiven Einheiten in einem Polymer bezeichnet man als Polymerisationsgrad. Erfindungsgemäß bevorzugte Polymere A wie auch Polymere B weisen jeweils einen Polymerisationsgrad im Bereich von 40 bis 1000, bevorzugt 100 bis 800, besonders bevorzugt 350 bis 650, auf. Weitere erfindungsgemäß bevorzugte Polymere A mit mindestens zehn konstitutiven Einheit der Formel (I) enthalten 40 bis 1000, bevorzugt 100 bis 800, besonders bevorzugt 350 bis 650 identische konstitutive Einheiten der Formel (I).

R¹ und R² stehen bevorzugt jeweils unabhängig voneinander für Wasserstoff oder für eine C₂-C₁₀-Acylgruppe, die bevorzugt ausgewählt aus einer Acetyl-, Propanoyl-, oder n-Butanoyl-Gruppe, besonders bevorzugt ausgewählt aus einer Acetyl-Gruppe.

Erfindungsgemäß bevorzugte Polymere A weisen mindestens 10 konstitutive Einheiten der Formel (I) auf, in denen X für Stickstoff steht, wobei das Polymer A keine permanent-ionischen konstitutiven Einheiten enthält.

Weitere erfindungsgemäß besonders bevorzugte Polymere A weisen mindestens 10 konstitutive Einheiten der Formel (I) auf, in denen X für Stickstoff steht und R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist.

Wenn R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, so ist dieser Ring bevorzugt mit mindestens einer funktionellen Gruppe substituiert, die ausgewählt ist aus =O. Ein besonders bevorzugte Substituenten-Kombination X, R¹, R² ist eine Pyrrolidon-Gruppe, so dass es sich bei einer erfindungsgemäß besonders bevorzugten konstitutiven Einheit der Formel (I) um eine Einheit der Formel (la) handelt, 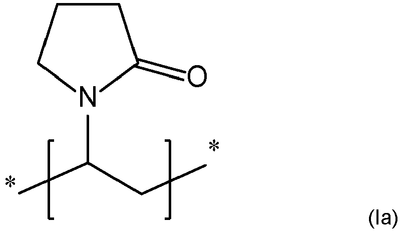 in der X für Stickstoff steht und R¹ und R² zusammen mit diesem Stickstoffatom einen fünfgliedrigen gesättigten Ring bilden, der keine weiteren Heteroatome enthält und der in 2-Position mit einer funktionellen Gruppe =O substituiert ist.

Eine weitere besonders bevorzugte Substituenten-Kombination X, R¹, R² ist eine ε-Caprolactam-Gruppe, so dass es sich bei einer erfindungsgemäß besonders bevorzugten konstitutiven Einheit der Formel (I) um eine Einheit der Formel (I b) handelt, 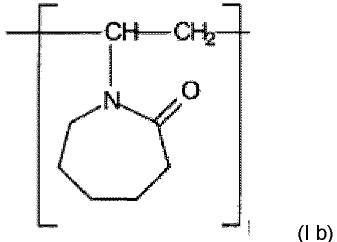 in der X für Stickstoff steht und R¹ und R² zusammen mit diesem Stickstoffatom einen sechsgliedrigen gesättigten Ring bilden, der keine weiteren Heteroatome enthält und der mit einer funktionellen Gruppe =O substituiert ist.

Eine weitere besonders bevorzugte Substituenten-Kombination X, R¹, R² ist eine Imidazol-Gruppe, so dass es sich bei einer weiteren erfindungsgemäß besonders bevorzugten Einheit der Formel (I) um eine Einheit handelt, in der X für Stickstoff steht und R¹ und R² zusammen mit diesem Stickstoffatom einen fünfgliedrigen ungesättigten Ring bilden, der als weiteres Heteroatom Stickstoff enthält.

Weitere erfindungsgemäß bevorzugte Polymere A weisen zu 25 - 100 Mol-%, bevorzugt zu 55 - 100 Mol-%, besonders bevorzugt zu 85 - 100 Mol-% konstitutive Einheiten der Formel (I) auf, in denen X für Stickstoff steht, wobei das Polymer A keine permanent-ionischen konstitutiven Einheiten enthält.

Weitere erfindungsgemäß bevorzugte Polymere A weisen zu 25 - 100 Mol-%, bevorzugt zu 55 - 100 Mol-%, besonders bevorzugt zu 85 - 100 Mol-% konstitutive Einheiten der Formel (I) auf, in denen X für Stickstoff steht und R¹ und R² zusammen mit X einen fünf- oder sechsgliedrigen, gesättigten oder ungesättigten Ring bilden, der gegebenenfalls weitere Heteroatome enthält, die ausgewählt sind aus N und O und gegebenenfalls mit mindestens einer C1 - C6-Alkylgruppe und/oder mit mindestens einer funktionellen Gruppe substituiert ist, wobei das Polymer A keine permanent-ionischen konstitutiven Einheiten enthält.

Erfindungsgemäß besonders bevorzugte Polymere A weisen zu 98 - 100 Mol-% konstitutive Einheiten der Formel (la) auf, wobei das Polymer A keine permanent-ionischen konstitutiven Einheiten enthält.

Erfindungsgemäß außerordentlich bevorzugte Polymere A weisen zu 98 - 100 Mol-% konstitutive Einheiten der Formel (la) auf und haben einen Polymerisationsgrad im Bereich von 40 bis 1000, bevorzugt 100 bis 800, besonders bevorzugt 350 bis 650, wobei das Polymer A keine permanent-ionischen konstitutiven Einheiten enthält. Besonders bevorzugte Polymere A sind Polyvinylpyrrolidon-Homopolymere mit einem Polymerisationsgrad im Bereich von 40 bis 1000, bevorzugt 100 bis 800, besonders bevorzugt 350 bis 650.

Eine weitere besonders bevorzugte Substituenten-Kombination X, R¹, R² ist eine konstitutive Einheit der Formel (I), in der X für Sauerstoff steht, p null ist und R¹ für Wasserstoff steht.

Eine weitere besonders bevorzugte Substituenten-Kombination X, R¹, R² ist eine konstitutive Einheit der Formel (I), in der X für Sauerstoff steht, p null ist und R¹ für eine Acetylgruppe steht. Weitere erfindungsgemäß bevorzugte Polymere A enthalten zu 75-92 Mol-% konstitutive Einheiten der Formel (I), in der X für Sauerstoff steht, p null ist und R¹ für Wasserstoff steht, und zu 8 - 25 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten 40 bis 1000, bevorzugt 100 bis 800, besonders bevorzugt 350 bis 650 konstitutive Einheiten der Formel (I), davon zu 75-92 Mol-% konstitutive Einheiten der Formel (I), in der X für Sauerstoff steht, p null ist und R¹ für Wasserstoff steht, und zu 8 - 25 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten zu 65-25 Mol-% konstitutive Einheiten der Formel (la) und zu 35 - 75 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten 40 bis 1000, bevorzugt 100 bis 800, besonders bevorzugt 350 bis 650 konstitutive Einheiten der Formel (I), davon zu 65-25 Mol-% konstitutive Einheiten der Formel (la) und zu 35 - 75 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Das mindestens eine Polymer A mit mindestens zehn konstitutiven Einheiten der Formel (I) weist keine permanenten ionischen Ladungen auf. Es ist aber möglich, dass die konstitutiven Einheiten der Formel (I), beispielsweise durch Protonierung des Stickstoffatoms in einem sauren Träger, ionisch, insbesondere kationisch, vorliegen. Diese Ladungen sind aber nicht permanent, sondern temporär, da sie vom umgebenden Medium abhängig sind.

Bevorzugte erfindungsgemäße Zusammensetzungen (A) enthalten das mindestens eine Polymer A mit mindestens zehn konstitutiven Einheiten der Formel (I) in einer Gesamtmenge von 0,5 bis 14 Gew.-%, bevorzugt 1,0 bis 11 Gew.-%, besonders bevorzugt 2,0 bis 10 Gew.-%, jeweils bezogen auf das Gewicht der Zusammensetzung (A).

### Permanent kationisches Polymer B (optional)

Als weiteren optionalen Inhaltsstoff enthalten erfindungsgemäß bevorzugte Zusammensetzungen (A) mindestens ein permanent kationisches Polymer B.

Bevorzugt enthält das permanent kationische Polymer neben mindestens einem permanent kationisch geladenen Monomer-Typ auch mindestens einen permanent anionisch geladenen Monomer-Typ, wobei die kationischen Monomere im molaren Überschuss zu den anionischen Monomeren vorliegen, so dass das mindestens eine zweite erfindungsgemäße Polymer eine kationische Nettoladung aufweist. Derartige erfindungsgemäß bevorzugte Polymere werden auch als amphotere oder zwitterionische Polymere bezeichnet.

In einer ersten bevorzugten Ausführungsform enthalten erfindungsgemäß bevorzugte Zusammensetzungen (A) mindestens ein permanent kationisches Polymer, das ausgewählt ist aus
- kationischen Polymeren, die aufgebaut sind aus Monomeren mit quartären Ammoniumgruppen der allgemeinen Formel (Ila),

   R³-CH=CR⁴-CO-Z-(CₙH₂ₙ)-N⁽⁺⁾R⁵R⁶R⁷ A⁽⁻⁾ (IIa),

   in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt,
   bevorzugt ausgewählt aus kationischen Polymeren, die aufgebaut sind aus Acrylamidopropyltrimethylammoniumchlorid,
   besonders bevorzugt ausgewählt aus amphoteren Polymeren mit kationischer Nettoladung, die durch Polymerisation aufgebaut sind aus
   c) kationischen Monomeren mit quartären Ammoniumgruppen der allgemeinen Formel (Ila),

      R³-CH=CR⁴-CO-Z-(CₙH₂ₙ)-N⁽⁺⁾R⁵R⁶R⁷ A⁽⁻⁾ (IIa),

      in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, und
   d) mindestens einer ungesättigten Carbonsäure, ausgewählt aus Acrylsäure, Methacrylsäure und Crotonsäure sowie aus Mischungen dieser Säuren, wobei die mindestens eine ungesättigte Carbonsäure in Form ihrer Salze vorliegen kann,
   wobei im Polymer die kationischen Monomere im molaren Überschuss zu den anionischen Monomeren vorliegen;
   außerordentlich bevorzugt ausgewählt aus amphoteren Polymeren mit kationischer Nettoladung, die den mindestens einen Monomer-Typ der allgemeinen Formel (Ila) und den
   mindestens einen Monomer-Typ der ungesättigten Carbonsäure, ausgewählt aus Acrylsäure, Methacrylsäure und Crotonsäure sowie Mischungen hiervon, in einem Molverhältnis von 60:40 bis 95:5, bevorzugt von 75:25 bis 90:10 zueinander enthalten,
   außerordentlichst bevorzugt ausgewählt aus amphoteren Copolymeren mit kationischer Nettoladung, die aus Acrylamidopropyltrimethylammoniumchlorid und Acrylsäure im Molverhältnis
   von 60:40 bis 95:5, bevorzugt von 75:25 bis 90:10 zueinander, bestehen;
- 2-[2-Hydroxy-3-(trimethylammonio)propoxy]ethylcelluloseetherchlorid, das beispielsweise unter der INCI-Bezeichnung Polyquaternium-10 erhältlich ist,
- Terpolymeren aus Acrylsäure, Diallyldimethylammoniumchlorid und Acrylamid, wie sie beispielsweise unter der INCI-Bezeichnung Polyquaternium-39 erhältlich sind,
- Homopolymeren des N,N,N-Trimethyl-2-[(methyl-1-oxo-2-propenyl)oxy]ethanaminiumchlorid, wie sie beispielsweise unter der INCI-Bezeichnung Polyquaternium-37 erhältlich sind,
- Copolymeren aus Diallyldimethylammoniumchlorid und Acrylsäure, wie sie beispielsweise unter der INCI-Bezeichnung Polyquaternium-22 erhältlich sind,
- Hydroxyethylcellulose-Dimethyldiallylammoniumchlorid-Copolymeren, wie sie beispielsweise unter der INCI-Bezeichnung Polyquaternium-4 erhältlich sind,
- Copolymeren aus Acrylamid und beta-Methacrylyloxyethyltrimethylammoniummethosulfat, wie sie beispielsweise unter der INCI-Bezeichnung Polyquaternium-5 erhältlich sind,
- Homopolymeren des N,N-Dimethyl-N-2-Propenyl-2-Propen-1-aminiumchlorid, wie sie beispielsweise unter der INCI-Bezeichnung Polyquaternium-6 erhältlich sind,
- Copolymeren aus Diallyldimethylammoniumchlorid und Acrylamid, wie sie beispielsweise unter der INCI-Bezeichnung Polyquaternium-7 erhältlich sind,
- Copolymeren aus Vinylpyrrolidon und Dimethylaminoethylmethacrylatdiethylsulfat, wie sie beispielsweise unter der INCI-Bezeichnung Polyquaternium-11 erhältlich sind,
- sowie Mischungen der vorgenannten Polymere.

Erfindungsgemäß außerordentlich bevorzugte permanente kationische Polymere sind ausgewählt aus 2-[2-Hydroxy-3-(trimethylammonio)propoxy]ethylcelluloseetherchlorid, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen und Terpolymeren aus Acrylsäure, Diallyldimethylammoniumchlorid und Acrylamid, sowie Zweier- und Dreier-Mischungen dieser Polymere.

Besonders bevorzugte Polymer B-Mischungen enthalten 2-[2-Hydroxy-3-(trimethylammonio)propoxy]ethylcelluloseetherchlorid und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten 2-[2-Hydroxy-3-(trimethylammonio)propoxy]ethylcelluloseetherchlorid, mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und mindestens ein Terpolymer aus Acrylsäure, Diallyldimethylammoniumchlorid und Acrylamid.

Erfindungsgemäß ebenfalls außerordentlich bevorzugte permanent-kationische Polymere B sind ausgewählt aus Polyquaternium-10, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen, und Polyquaternium-39, sowie Zweier- und Dreier-Mischungen dieser Polymere.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39.

Bevorzugte erfindungsgemäße Zusammensetzungen (A) enthalten das mindestens eine permanent kationische Polymer B in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A).

Weitere erfindungsgemäß bevorzugte Zusammensetzungen (A) enthalten mindestens ein permanent kationisches Polymer B, ausgewählt aus
- kationischen Polymeren, die aufgebaut sind aus Monomeren mit quartären Ammoniumgruppen der allgemeinen Formel (Ila),

   R³-CH=CR⁴-CO-Z-(CₙH₂ₙ)-N⁽⁺⁾R⁵R⁶R⁷ A⁽⁻⁾ (IIa),

   in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt,
   bevorzugt ausgewählt aus kationischen Polymeren, die aufgebaut sind aus Acrylamidopropyltrimethylammoniumchlorid,
   besonders bevorzugt ausgewählt aus amphoteren Polymeren mit kationischer Nettoladung, die durch Polymerisation aufgebaut sind aus
   e) kationischen Monomeren mit quartären Ammoniumgruppen der allgemeinen Formel (Ila),

      R³-CH=CR⁴-CO-Z-(CₙH₂ₙ)-N⁽⁺⁾R⁵R⁶R⁷ A⁽⁻⁾ (IIa),

      in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, und
   f) mindestens einer ungesättigten Carbonsäure, ausgewählt aus Acrylsäure, Methacrylsäure und Crotonsäure sowie aus Mischungen dieser Säuren, wobei die mindestens eine ungesättigte Carbonsäure in Form ihrer Salze vorliegen kann,
   wobei im Polymer die Gesamtheit aller kationischen Monomere im molaren Überschuss zur Gesamtheit aller anionischen Monomere vorliegt;
   außerordentlich bevorzugt ausgewählt aus amphoteren Polymeren mit kationischer Nettoladung, die den mindestens einen Monomer-Typ der allgemeinen Formel (IIa) und den mindestens einen Monomer-Typ der ungesättigten Carbonsäure, ausgewählt aus Acrylsäure, Methacrylsäure und Crotonsäure sowie Mischungen hiervon, in einem Molverhältnis von 60:40 bis 95:5, bevorzugt von 75:25 bis 90:10 zueinander enthalten,
   außerordentlichst bevorzugt ausgewählt aus amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen;
- 2-[2-Hydroxy-3-(trimethylammonio)propoxy]ethylcelluloseetherchlorid, das beispielsweise unter der INCI-Bezeichnung Polyquaternium-10 erhältlich ist,
- Terpolymeren aus Acrylsäure, Diallyldimethylammoniumchlorid und Acrylamid, wie sie beispielsweise unter der INCI-Bezeichnung Polyquaternium-39 erhältlich sind,
- Homopolymeren des N,N,N-Trimethyl-2-[(methyl-1-oxo-2-propenyl)oxy]ethanaminiumchlorid, wie sie beispielsweise unter der INCI-Bezeichnung Polyquaternium-37 erhältlich sind,
- Copolymeren aus Diallyldimethylammoniumchlorid und Acrylsäure, wie sie beispielsweise unter der INCI-Bezeichnung Polyquaternium-22 erhältlich sind,
- Hydroxyethylcellulose-Dimethyldiallylammoniumchlorid-Copolymeren, wie sie beispielsweise unter der INCI-Bezeichnung Polyquaternium-4 erhältlich sind,
- Copolymeren aus Acrylamid und beta-Methacrylyloxyethyltrimethylammoniummethosulfat, wie sie beispielsweise unter der INCI-Bezeichnung Polyquaternium-5 erhältlich sind,
- Homopolymeren des N,N-Dimethyl-N-2-Propenyl-2-Propen-1-aminiumchlorid, wie sie beispielsweise unter der INCI-Bezeichnung Polyquaternium-6 erhältlich sind,
- Copolymeren aus Diallyldimethylammoniumchlorid und Acrylamid, wie sie beispielsweise unter der INCI-Bezeichnung Polyquaternium-7 erhältlich sind, sowie
- Copolymeren aus Vinylpyrrolidon und Dimethylaminoethylmethacrylatdiethylsulfat, wie sie beispielsweise unter der INCI-Bezeichnung Polyquaternium-11 erhältlich sind,
in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A).

Weitere erfindungsgemäß bevorzugte Zusammensetzungen (A) enthalten 2-[2-Hydroxy-3-(trimethylammonio)propoxy]ethylcelluloseetherchlorid und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A). Weitere erfindungsgemäß bevorzugte Zusammensetzungen (A) enthalten 2-[2-Hydroxy-3-(trimethylammonio)propoxy]ethylcelluloseetherchlorid, mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und mindestens ein Terpolymer aus Acrylsäure, Diallyldimethylammoniumchlorid und Acrylamid, in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A). Weitere besonders bevorzugte Zusammensetzungen (A) enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A).

Weitere besonders bevorzugte Zusammensetzungen (A) enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39 in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A).

Optional kann die vorgenannte Zusammensetzung (A) weiterhin mindestens eine Substanz enthalten, die ausgewählt ist aus Verbindungen der allgemeinen Formel (III), 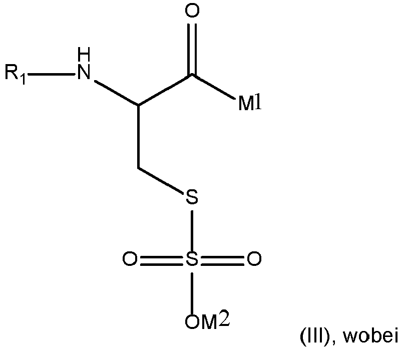
R1 für ein Wasserstoffatom oder für ein Strukturelement der Formel (IV) steht 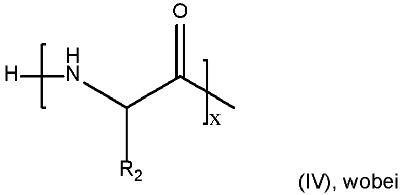
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (V) 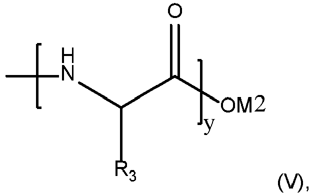
wobei
y für eine ganze Zahl von 1 bis 100 steht,
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺.

In einer ganz besonders bevorzugten Ausführungsform ist eine erfindungsgemäße Zusammensetzung (A) dadurch gekennzeichnet, dass sie mindestens eine Verbindung der Formel (III) enthält, die ein Molekulargewicht M_{w} von 200 bis 2.000 Da (Dalton), vorzugsweise von 250 bis 1.500 Da, bevorzugt von 300 bis 1.200 Da, insbesondere von 400 bis 800 Da aufweist.

Wird in der erfindungsgemäßen Zusammensetzung (A) eine Mischung von Oligomeren eingesetzt, so können diese Mischungen durch ihr mittleres Molekulargewicht definiert werden.

In diesem Fall ist eine erfindungsgemäß bevorzugte Zusammensetzung (A) dadurch gekennzeichnet, dass sie mindestens eine Mischung von Verbindungen der Formel (III) enthält, die ein mittleres Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Weiterhin hat sich gezeigt, dass der Schutz- oder Repair-Effekt, den die Verbindungen der Formel (III) besitzen, auch von den Wiederholungsindices x und y abhängt. Wie zuvor beschrieben, ist es ganz besonders bevorzugt, wenn x für eine ganze Zahl von 1 bis 10 steht und y für eine ganze Zahl von 1 bis 10 steht.

In einer weiteren ganz besonders bevorzugten Ausführungsform ist eine erfindungsgemäße Zusammensetzung (A) dadurch gekennzeichnet, dass sie mindestens eine Verbindung der Formel (III) enthält, wobei
R1 für ein Strukturelement der Formel (IV) steht, und
M1 für ein Strukturelement der Formel (V) steht, und
x für eine ganze Zahl von 1 bis 10 steht und
y für eine ganze Zahl von 1 bis 10 steht.

Neben dem Molekulargewicht der Verbindung der Formel (III) nimmt auch der Anteil der Bunte-Salz Einheiten, die in der Verbindung der Formel (III) enthalten sind, einen entscheidenden Einfluss auf die Wirksamkeit der Schutzwirkung oder "Repair-Wirkung" der Verbindungen. Verbindungen mit mindestens einer Bunte-Salz-Einheit - wie sie beispielsweise in der Verbindung der Formel (Illa) vorhanden ist - sind sehr effektiv, insbesondere wenn sie als monomere Verbindung eingesetzt werden. Oligopeptide mit mindestens einer Bunte-Salz Einheit sind besonders wirksam, wenn sie ein niedriges Molekulargewicht von bis zu 1200, insbesondere 800 Dalton besitzen.

Bei Einsatz von Oligopeptiden ist es jedoch von ganz besonderem Vorteil, wenn die Verbindung der Formel (III) mindestens zwei, bevorzugt mindestens drei Bunte-Salz Einheiten besitzt.

In einer weiteren ganz besonders bevorzugten Ausführungsform ist eine erfindungsgemäße Zusammensetzung (A) dadurch gekennzeichnet, dass sie mindestens eine Verbindung der Formel (III) enthält, wobei
R1 für ein Strukturelement der Formel (IV) steht, und
der Rest R2 in mindestens einem Strukturelement der Formel (IV) für eine
(Sulfosulfanyl)methyl-Gruppe (d.h. eine Gruppe HO-S(O₂)-S-CH₂-) steht.

In einer weiteren ganz besonders bevorzugten Ausführungsform ist eine erfindungsgemäße Zusammensetzung (A) dadurch gekennzeichnet, dass sie mindestens eine Verbindung der Formel (III) enthält, wobei
R1 für ein Strukturelement der Formel (IV) steht, und
x für eine ganze Zahl von mindestens 3 steht und
der Rest R2 in mindestens 3 Strukturelementen der Formel (IV) für eine 2-Carboxyethyl-Gruppe (d.h. eine Gruppe HOOC-CH2-CH2-) steht.

In einer weiteren ganz besonders bevorzugten Ausführungsform ist eine erfindungsgemäße Zusammensetzung (A) dadurch gekennzeichnet, dass sie mindestens eine Verbindung der Formel (III) enthält, wobei
M1 für ein Strukturelement der Formel (V) steht, und y für eine ganze Zahl von mindestens 3 steht und
der Rest R3 in mindestens 3 Strukturelementen der Formel (IV) für eine Gruppe (Glu) steht. Die mindestens eine Verbindung der Formel (III) ist - bezogen auf das Gesamtgewicht der erfindungsgemäß bevorzugten Zusammensetzung (A) in einer Gesamtmenge von 0,001 bis 10 Gew.-% enthalten. Überraschenderweise hat sich jedoch herausgestellt, dass die Verbindung(en) der Formel (III) bereits in geringen Einsatzkonzentrationen eine sehr gute Reduzierung der Haarschädigung bewirken können. Dies ist insbesondere deshalb von Vorteil, weil die mindestens eine Verbindung der Formel (III) in der erfindungsgemäßen Zusammensetzung (A) als Additiv (beispielsweise in Form einer Pflegelösung oder Reparaturlösung) vor der Applikation auf das Haar hinzugefügt werden soll. Aus diesem Grund ist es besonders vorteilhaft, wenn die erfindungsgemäß bevorzugte Zusammensetzung (A) eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht der erfindungsgemäßen Zusammensetzung (A).

In einer weiteren ganz besonders bevorzugten Ausführungsform ist eine erfindungsgemäße Zusammensetzung (A) dadurch gekennzeichnet, dass sie eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht der erfindungsgemäßen Zusammensetzung (A), wobei in Formel (III) R1 für ein Strukturelement der Formel (IV) steht, und der Rest R2 in mindestens einem Strukturelement der Formel (IV) für eine (Sulfosulfanyl)methyl-Gruppe (d.h. eine Gruppe HO-S(O₂)-S-CH₂-) steht.

Weiterhin kann es erfindungsgemäß bevorzugt sein, die Haar schützende erfindungsgemäße Kombination aus mindestens einer gesättigten Dicarbonsäure mit 2 bis 10 Kohlenstoffatomen und mindestens einer Aminosäure der Formel (VI) zunächst zusammen mit der Zubereitung, die das mindestens eine Alkalisierungsmittel und ggf. mindestens ein Oxidationsfarbstoffvorprodukt und/oder mindestens einen direktziehenden Farbstoff enthält, aber getrennt von der Oxidationsmittelzubereitung, die mindestens eine Peroxidverbindung enthält, aufzubewahren und erst kurz vor Beginn des erfindungsgemäßen oder erfindungsgemäß bevorzugten Verfahrens zur oxidativen Färbung und/oder Aufhellung der keratinischen Fasern das erfindungsgemäße oder erfindungsgemäß bevorzugte Färbe- oder Blondiermittel durch Vermischen der beiden Komponenten herzustellen.

In einer weiteren ganz besonders bevorzugten Ausführungsform weist die erfindungsgemäße Zusammensetzung (A) einen pH-Wert im Bereich von 3,5 bis 7,1, bevorzugt 4,5 - 6,5, besonders bevorzugt 5,0 bis 6,0, auf, jeweils gemessen bei 20°C.

Ein weiterer Gegenstand der vorliegenden Anmeldung ist daher ein Verfahren zur oxidativen Färbung und/oder Aufhellung keratinischer Fasern, insbesondere von Humanhaar, das folgende Verfahrensschritte umfasst:
I. Bereitstellen einer Zusammensetzung (AB), enthaltend
   a) mindestens eine Dicarbonsäure mit 2 bis 10 Kohlenstoffatomen, ausgewählt aus Maleinsäure und Fumarsäure, und/oder mindestens ein Salz dieser Säure(n),
   b) mindestens eine Aminosäure der Formel (VI), 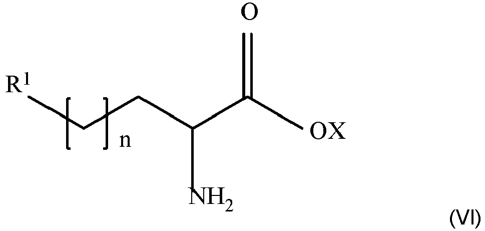 worin
      X für ein Wasserstoffatom oder ein ein- oder zweiwertiges Kation steht;
      n für null, 1, 2 oder 3 steht;
      R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃ oder mindestens ein Salz dieser Aminosäure,
      wobei die Aminosäure der Formel (VI) bevorzugt ausgewählt ist aus Arginin, Lysin, Histidin sowie Mischungen hiervon, besonders bevorzugt Mischungen aus Arginin und Lysin, oder mindestens einem Salz dieser Aminosäuren,
   c) optional weiterhin mindestens eine Substanz, die ausgewählt ist aus
      - Verbindungen der allgemeinen Formel (III), 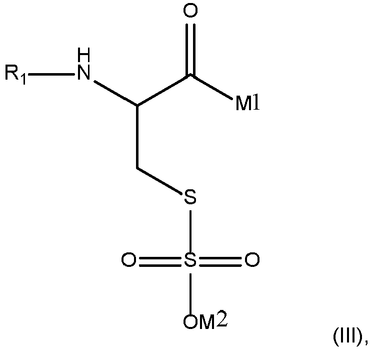 wobei
         R1 für ein Wasserstoffatom oder für ein Strukturelement der Formel (IV) steht 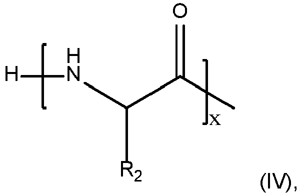
         wobei
         x für eine ganze Zahl von 1 bis 100 steht,
         der Rest R2 in jedem der Strukturelemente der Formel (IV) jeweils unabhängig vom vorangegangenen Strukturelement der Formel (IV) gewählt werden kann,
         R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
         M1 steht für die Gruppierung -OM2 oder für ein Strukturelement der Formel (V) 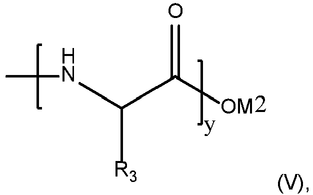
         wobei
         y für eine ganze Zahl von 1 bis 100 steht,
         der Rest R3 in jedem der Strukturelemente der Formel (V) jeweils unabhängig vom vorangegangenen Strukturelement der Formel (V) gewählt werden kann,
         R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
         M2 steht für ein Wasserstoffatom, ein Äquivalent eines ein oder mehrwertigen Kations oder ein Ammoniumion (NH₄)⁺, und
      - Polymeren A, die mindestens zehn konstitutive Einheiten der Formel (I) aufweisen, 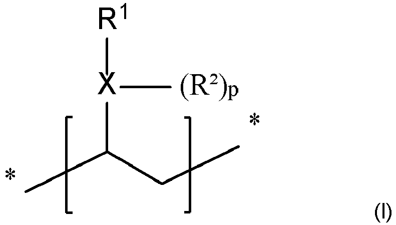 worin
      - X für Stickstoff oder Sauerstoff steht und
      - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
      - p = 0 ist, wenn X für Sauerstoff steht und p = 1 ist, wenn X für Stickstoff steht, wobei das Polymer A keine permanent-ionischen konstitutiven Einheiten enthält,
   d) mindestens ein Alkalisierungsmittel, ausgewählt aus Ammoniumhydroxid, Monoethanolamin und Natriumsilikaten sowie Mischungen hiervon,
   e) Wasser und
   f) ggf. mindestens ein Oxidationsfarbstoffvorprodukt und/oder mindestens einen direktziehenden Farbstoff,
II. Bereitstellen einer Zusammensetzung (C), enthaltend
   g) mindestens eine Peroxidverbindung, die bevorzugt Wasserstoffperoxid ist,
III. Vermischen der Zusammensetzungen (AB) und (C) miteinander, direkt anschließend
IV. Auftragen der Mischung aus (AB) und (C) auf die keratinischen Fasern, insbesondere auf das Humanhaar, und
V. Ausspülen nach einer Einwirkzeit von 0,1 bis 60 Minuten, bevorzugt 1 bis 45 Minuten, besonders bevorzugt 10 bis 30 Minuten,
VI. ggf. weitere Haarbehandlungen, wie Verformen, Konditionieren und/oder Trocknen.

### Mischungsverhältnisse der Zusammensetzung (AB) mit Zusammensetzung (C)

Es hat sich erfindungsgemäß bewährt, wenn das Gewichtsverhältnis aus der Zusammensetzung (AB), die die Haar schützende Kombination aus mindestens einer Dicarbonsäure mit 2 bis 10 Kohlenstoffatomen, ausgewählt aus Maleinsäure und Fumarsäure, und/oder mindestens ein Salz dieser Säure(n), und mindestens einer Aminosäure der Formel (VI) und Wasser in der alkalisierenden Zusammensetzung (B), enthaltend mindestens ein Alkalisierungsmittel, ausgewählt aus Ammoniumhydroxid, Monoethanolamin und Natriumsilikaten sowie Mischungen hiervon, und ggf. Oxidationsfarbstoffvorprodukte und/oder direktziehende Farbstoffe enthält und der Zusammensetzung (C), enthaltend mindestens eine Peroxidverbindung, im Bereich von [Gewicht von AB]/[[Gewicht von C] wie 1:0,8 bis 1:2,5, bevorzugt 1:1 bis 1:2 liegt.

Erfindungsgemäß bevorzugte Färbe- oder Aufhellverfahren mit den mindestens zwei vorgenannten Zusammensetzungen (AB) und (C) sind dadurch gekennzeichnet, dass die mindestens eine Dicarbonsäure mit 2 bis 10 Kohlenstoffatomen, ausgewählt aus Maleinsäure und Fumarsäure, und/oder mindestens ein Salz dieser Säure(n), in der Zusammensetzung (AB) in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht der Zusammensetzung (AB), enthalten ist, wobei die Gesamtmenge an Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, beträgt jeweils bezogen auf das Gewicht der Zusammensetzung (AB).

Erfindungsgemäß bevorzugte Färbe- oder Aufhellverfahren mit den mindestens zwei vorgenannten Zusammensetzungen (AB) und (C) sind weiterhin dadurch gekennzeichnet, dass die mindestens eine Aminosäure der Formel (VI) in der Zusammensetzung (AB) in einer auf die Masse an freier Aminosäure umgerechneten Gesamtmenge von 0,1 - 7 Gew.-%, bevorzugt 0,2 - 5 Gew.-%, besonders bevorzugt 0,3 - 2,5 Gew.-%, außerordentlich bevorzugt 1 - 2 Gew.-%, jeweils bezogen auf das Gewicht der Zusammensetzung (AB), enthalten ist.

Weitere erfindungsgemäß bevorzugte Färbe- oder Aufhellverfahren mit den mindestens zwei vorgenannten Zusammensetzungen (AB) und (C) sind dadurch gekennzeichnet, dass in der Zusammensetzung (AB) eine oder mehrere Verbindungen der vorstehend aufgeführten Formel (III) in einer Gesamtmenge von 0,002 bis 2,5 Gew.-% weiter bevorzugt von 0,02 bis 2,0 Gew.-% und besonders bevorzugt von 0,04 bis 0,2 Gew.-% enthalten sind, jeweils bezogen auf das Gewicht der Zusammensetzung (AB), enthalten sind.

Weitere erfindungsgemäß bevorzugte Färbe- oder Aufhellverfahren mit den mindestens zwei vorgenannten Zusammensetzungen (AB) und (C) sind dadurch gekennzeichnet, dass die Zusammensetzung (C) 1,0 bis 23,0 Gew.-%, weiter bevorzugt 2,5 bis 21,0 Gew.-%, besonders bevorzugt 4,0 bis 20,0 Gew.-% und ganz besonders bevorzugt 5,0 bis 18,0 Gew.-% Wasserstoffperoxid (berechnet als 100 %-iges HzOz) enthält, jeweils bezogen auf das Gewicht der Zusammensetzung (C).

Die erfindungsgemäßen Zusammensetzungen (AB) enthalten Wasser, und zwar bevorzugt in einer

Menge von 20 bis 85 Gew.-%, bevorzugt 30 bis 80 Gew.-%, jeweils bezogen auf das Gesamtgewicht der erfindungsgemäßen Zusammensetzung (AB).

Die erfindungsgemäßen Zusammensetzungen (AB) weisen bevorzugt einen pH-Wert im Bereich von 6,5 bis 10,5, bevorzugt 8 - 10, besonders bevorzugt 8,5 bis 0,5, aufweist, jeweils gemessen bei 20°C.

Für das erfindungsgemäße Verfahren zur oxidativen Färbung und/oder Aufhellung keratinischer Fasern, insbesondere von Humanhaar, mit dem aus den mindestens zwei vorstehend genannten Zusammensetzungen (AB) und (C) und seine bevorzugten Ausführungsformen gilt - abgesehen von den geänderten quantitativen Angaben, die vorstehend genannt sind - mutatis mutandis das zu den erfindungsgemäßen und erfindungsgemäß bevorzugten Färbe- und Blondiermitteln Gesagte.

## Patentansprüche

1. Oxidatives Färbe- oder Blondiermittel für keratinische Fasern, insbesondere für Humanhaar, enthaltend
a) mindestens eine Dicarbonsäure mit 2 bis 10 Kohlenstoffatomen, ausgewählt aus Maleinsäure und Fumarsäure, und/oder mindestens ein Salz dieser Säure(n),
b) mindestens eine Aminosäure der Formel (VI) 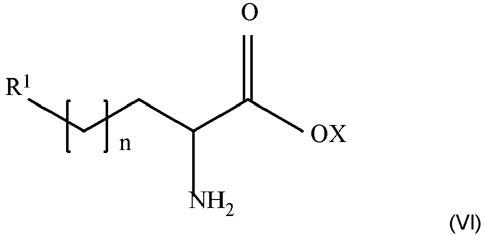 worin
X für ein Wasserstoffatom oder ein ein- oder zweiwertiges Kation steht;
n für null, 1, 2 oder 3 steht;
R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
c) weiterhin mindestens ein Alkalisierungsmittel, ausgewählt aus Ammoniumhydroxid, Monoethanolamin und Natriumsilikaten sowie Mischungen hiervon,
d) ggf. mindestens ein Oxidationsfarbstoffvorprodukt und/oder mindestens einen direktziehenden Farbstoff,
e) Wasser und
f) mindestens eine Peroxidverbindung.

2. Färbe- oder Blondiermittel gemäß Anspruch 1, **dadurch gekennzeichnet, dass** die mindestens eine Dicarbonsäure mit 2 bis 10 Kohlenstoffatomen in einer Gesamtmenge von 0,2 bis 4 Gew.-%, bevorzugt 0,33 bis 3 Gew.-%, besonders bevorzugt 0,5 bis 2 Gew.-%, enthalten ist, jeweils umgerechnet auf die undissoziierte Dicarbonsäure und bezogen auf das Gewicht des Färbe- oder Blondiermittels, wobei die Gesamtmenge an Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure 0,2 bis 4 Gew.-%, bevorzugt 0,33 bis 3 Gew.-%, besonders bevorzugt 0,5 bis 2 Gew.-%, beträgt, jeweils umgerechnet auf die undissoziierte Dicarbonsäure und bezogen auf das Gewicht des Färbe- oder Blondiermittels.

3. Färbe- oder Blondiermittel gemäß einem der Ansprüche 1 oder 2, **dadurch gekennzeichnet, dass** die mindestens eine Aminosäure der Formel (VI) ausgewählt ist aus Arginin, Lysin, Histidin, Asparagin, Glutamin, Cystein, Methionin, Tryptophan sowie Mischungen hiervon, besonders bevorzugt Mischungen aus Arginin und Lysin.

4. Färbe- oder Blondiermittel gemäß einem der Ansprüche 1 bis 3, **dadurch gekennzeichnet, dass** die mindestens eine Aminosäure der Formel (VI) in einer Gesamtmenge von 0,05 bis 3 Gew.-%, bevorzugt 0,1 bis 2 Gew.-%, besonders bevorzugt 0,2 bis 1,2 Gew.-%, enthalten ist, jeweils umgerechnet auf die undissoziierte Aminosäure und bezogen auf das Gewicht des Färbe- oder Blondiermittels.

5. Färbe- oder Blondiermittel gemäß einem der Ansprüche 1 bis 4, **dadurch gekennzeichnet, dass** Mischungen aus Arginin und Lysin oder mindestens einem Salz dieser Aminosäuren in einer Gesamtmenge von 0,05 bis 3 Gew.-%, bevorzugt 0,1 bis 2 Gew.-%, besonders bevorzugt 0,2 bis 1,2 Gew.-%, enthalten sind, jeweils umgerechnet auf die undissoziierte Aminosäure und bezogen auf das Gewicht des Färbe- oder Blondiermittels.

6. Färbe- oder Blondiermittel gemäß einem der Ansprüche 1 - 5, **dadurch gekennzeichnet, dass**
mindestens eine Verbindung der allgemeinen Formel (III) enthalten ist, 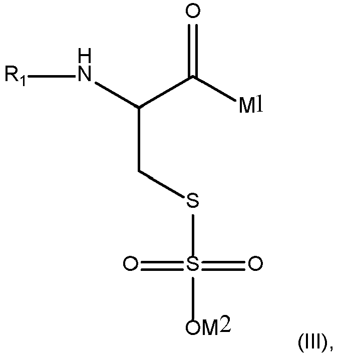
wobei
R1 für ein Wasserstoffatom oder für ein Strukturelement der Formel (IV) steht 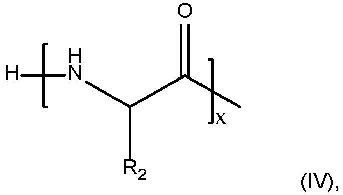
wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (V) 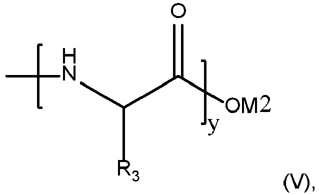
wobei
y für eine ganze Zahl von 1 bis 100 steht,
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺,
wobei bevorzugt eine oder mehrere Verbindungen der vorstehend aufgeführten Formel (III) in einer Gesamtmenge von 0,001 bis 2,5 Gew.-% weiter bevorzugt von 0,01 bis 1,0 Gew.-
% und besonders bevorzugt von 0,02 bis 0,1 Gew.-% enthalten ist, jeweils bezogen auf das Gewicht des erfindungsgemäßen Färbe- oder Blondiermittels.

7. Färbe- oder Blondiermittel gemäß einem der Ansprüche 1 - 6, **dadurch gekennzeichnet, dass** 20 bis 85 Gew.-%, bevorzugt 30 bis 80 Gew.-%, Wasser enthalten ist, jeweils bezogen auf das Gesamtgewicht des erfindungsgemäßen Färbe- oder Blondiermittels.

8. Färbe- oder Blondiermittel gemäß einem der Ansprüche 1 - 7, **dadurch gekennzeichnet, dass** als Peroxidverbindung Wasserstoffperoxid enthalten ist.

9. Färbe- oder Blondiermittel gemäß einem der Ansprüche 1 - 8, **dadurch gekennzeichnet, dass** 0,5 bis 13 Gew.-%, weiter bevorzugt 1 bis 7 Gew.-%, besonders bevorzugt 2 bis 6 Gew.-% und ganz besonders bevorzugt 3 bis 4,5 Gew.-% Wasserstoffperoxid (berechnet als 100 %-iges HzOz) enthalten ist, jeweils bezogen auf das Gesamtgewicht des erfindungsgemäßen Färbe- oder Blondiermittels.

10. Färbe- oder Blondiermittel gemäß einem der Ansprüche 1 - 9, **dadurch gekennzeichnet, dass** mindestens ein Polymer A enthalten ist, das mindestens zehn konstitutive Einheiten der Formel
(I) aufweist, 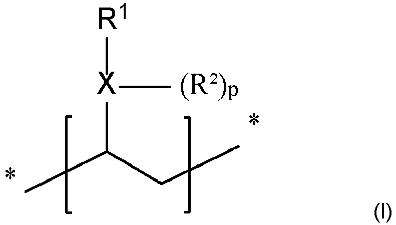 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht, wobei das Polymer A keine permanent-ionischen konstitutiven Einheiten enthält,
wobei das mindestens eine Polymer A mit mindestens zehn konstitutiven Einheiten der Formel (I) bevorzugt in einer Gesamtmenge von 0,2 bis 5 Gew.-%, besonders bevorzugt 0,5 bis 3 Gew.-%, außerordentlich bevorzugt 1,0 bis 2,3 Gew.-%, jeweils bezogen auf das Gewicht des Färbe- oder Blondiermittels, enthalten ist.

11. Färbe- oder Blondiermittel gemäß Anspruch 10, **dadurch gekennzeichnet, dass** das mindestens eine Polymer A mit mindestens zehn konstitutiven Einheiten der Formel (I) ausgewählt ist aus Polymeren, die zu 98 - 100 Mol-% konstitutive Einheiten der Formel (la) 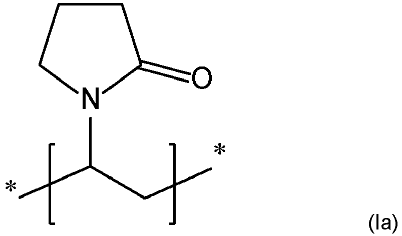 aufweisen und einen Polymerisationsgrad im Bereich von 40 bis 1000, bevorzugt 100 bis 800, besonders bevorzugt 350 bis 650 haben, wobei das Polymer A keine permanent-ionischen konstitutiven Einheiten enthält.

12. Verfahren zur oxidativen Färbung und/oder Aufhellung keratinischer Fasern, insbesondere von Humanhaar, bei dem ein Färbe- oder Blondiermittel gemäß einem der Ansprüche 1 - 11 auf die keratinischen Fasern, insbesondere auf das Humanhaar, aufgetragen und nach einer Einwirkzeit von 0,1 bis 60 Minuten, bevorzugt 1 bis 45 Minuten, besonders bevorzugt 10 bis 30 Minuten, wieder ausgespült wird.

13. Verfahren zur oxidativen Färbung und/oder Aufhellung keratinischer Fasern, insbesondere von Humanhaar, das folgende Verfahrensschritte umfasst:
I. Bereitstellen einer Zusammensetzung (A), enthaltend
a) mindestens eine Dicarbonsäure mit 2 bis 10 Kohlenstoffatomen, ausgewählt aus Maleinsäure und Fumarsäure, und/oder mindestens ein Salz dieser Säure(n),
b) mindestens eine Aminosäure der Formel (VI) 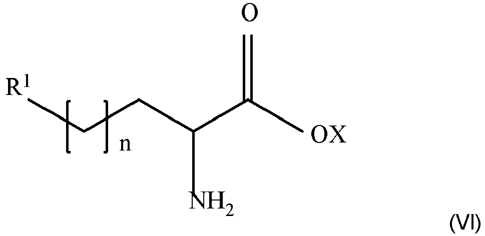 worin
X für ein Wasserstoffatom oder ein ein- oder zweiwertiges Kation steht; n für null, 1, 2 oder 3 steht;
R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
c) Wasser und
d) optional weiterhin mindestens eine Substanz, die ausgewählt ist aus
- Verbindungen der allgemeinen Formel (III), 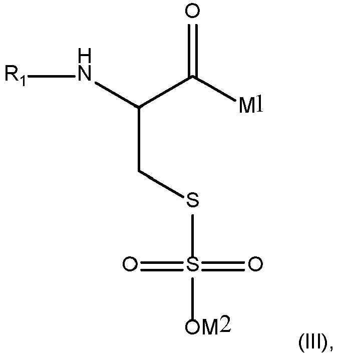 wobei
R1 für ein Wasserstoffatom oder für ein Strukturelement der Formel (IV) steht 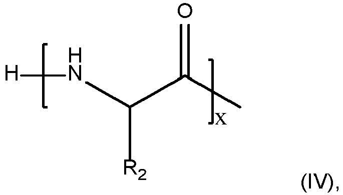
wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (V) 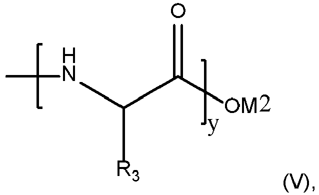
wobei
y für eine ganze Zahl von 1 bis 100 steht,
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺, und
- Polymeren A, die mindestens zehn konstitutive Einheiten der Formel (I) aufweisen, 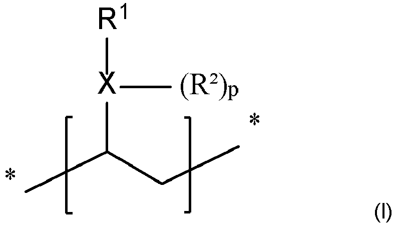 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält, wobei die Zusammensetzung (A) bevorzugt einen pH-Wert im Bereich von 3,5 bis 7,1, bevorzugt 4,5 - 6,5, besonders bevorzugt 5,0 bis 6,0, aufweist, jeweils gemessen bei 20°C,
II. Bereitstellen einer Zusammensetzung (B), enthaltend
e) mindestens ein Alkalisierungsmittel, ausgewählt aus Ammoniumhydroxid, Monoethanolamin und Natriumsilikaten sowie Mischungen hiervon,
f) ggf. Wasser und
g) ggf. mindestens ein Oxidationsfarbstoffvorprodukt und/oder mindestens einen direktziehenden Farbstoff,
III. Bereitstellen einer Zusammensetzung (C), enthaltend
h) mindestens eine Peroxidverbindung, die bevorzugt Wasserstoffperoxid ist,
IV. Vermischen der Zusammensetzungen (A), (B) und (C) miteinander, direkt anschließend
V. Auftragen der Mischung aus (A), (B) und (C) auf die keratinischen Fasern, insbesondere auf das Humanhaar, und
VI. Ausspülen nach einer Einwirkzeit von 0,1 bis 60 Minuten, bevorzugt 1 bis 45 Minuten, besonders bevorzugt 10 bis 30 Minuten,
VII. ggf. weitere Haarbehandlungen, wie Verformen, Konditionieren und/oder Trocknen.

14. Zusammensetzung (A), enthaltend
- mindestens eine Dicarbonsäure mit 2 bis 10 Kohlenstoffatomen, ausgewählt aus Maleinsäure und Fumarsäure, in einer Gesamtmenge von 2 bis 20 Gew.-%, bevorzugt 5 bis 15 Gew.-%, besonders bevorzugt 8 bis 12 Gew.-%, jeweils umgerechnet auf die undissoziierte Dicarbonsäure und bezogen auf das Gewicht der Zusammensetzung (A), wobei die Gesamtmenge an Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure 2 bis 20 Gew.-%, bevorzugt 5 bis 15 Gew.-%, besonders bevorzugt 8 bis 12 Gew.-%, beträgt, jeweils umgerechnet auf die undissoziierte Dicarbonsäure und bezogen auf das Gewicht der Zusammensetzung (A), weiterhin
- mindestens eine Aminosäure der Formel (VI) und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), wobei bevorzugt mindestens eine der Aminosäuren Arginin, Histidin oder Lysin und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A) enthalten ist, und
- Wasser, bevorzugt in einer Menge von 50 - 92 Gew.-%, besonders bevorzugt 60 - 87 Gew.-% und außerordentlich bevorzugt von 65 bis 80 Gew.-%, jeweils bezogen auf das Gewicht der Zusammensetzung (A),
- optional weiterhin mindestens ein Polymer A, das mindestens zehn konstitutive Einheiten der Formel (I) aufweist, 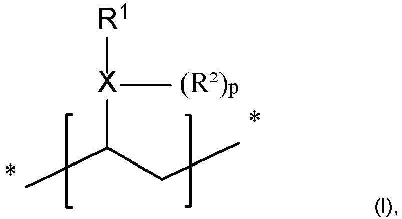 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht, wobei das Polymer A keine permanent-ionischen konstitutiven Einheiten enthält, wobei bevorzugt das mindestens eine Polymer A in einer Gesamtmenge von 0,5 bis 14 Gew.-%, bevorzugt 1,0 bis 11 Gew.-%, besonders bevorzugt 2,0 bis 10 Gew.-%, jeweils bezogen auf das Gewicht der Zusammensetzung (A), enthalten ist,
wobei die Zusammensetzung (A) bevorzugt einen pH-Wert im Bereich von 3,5 bis 7,1, bevorzugt 4,5 - 6,5, besonders bevorzugt 5,0 bis 6,0, aufweist, jeweils gemessen bei 20°C.

## Claims

1. Oxidative dyeing or bleaching agent for keratin fibers, in particular for human hair, containing
a) at least one dicarboxylic acid with 2 to 10 carbon atoms, selected from maleic acid and fumaric acid, and/or at least one salt of these acid(s),
b) at least one amino acid of the formula (VI) 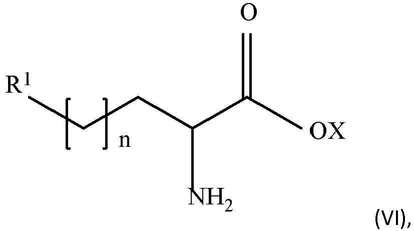 where
X represents a hydrogen atom or a mono- or divalent cation;
n represents zero, 1, 2 or 3;
R1 represents a residue which is selected from an amino group, a guanidine group, a (1H-imidazol-4-yl) group, a carboxamide group -CONH2, a 1H-indol-3-yl group, a thiol group -SH and a methylsulfanyl group -SCH3, or at least one salt of this amino acid,
c) further at least one alkalizing agent selected from ammonium hydroxide, monoethanolamine and sodium silicates and mixtures thereof,
d) optionally at least one oxidation dye precursor and / or at least one substantive dye,
e) water and
f) at least one peroxide compound.

2. Dyeing or bleaching agent according to claim 1, **characterized in that** the at least one dicarboxylic acid with 2 to 10 carbon atoms is contained in a total amount of 0.2 to 4% by weight, preferably 0.33 to 3% by weight, particularly preferably 0.5 to 2% by weight, in each case converted to undissociated dicarboxylic acid and based on the weight of the dyeing or bleaching agent, wherein the total amount of malic acid, D-tartaric acid, L-tartaric acid, meso-tartaric acid, grape acid, alpha-ketoglutaric acid, beta-ketoglutaric acid, oxaloacetic acid, maleic acid and fumaric acid is 0.2 to 4% by weight, preferably 0.33 to 3% by weight, particularly preferably 0.5 to 2% by weight, in each case converted to undissociated dicarboxylic acid and based on the weight of the dyeing or bleaching agent.

3. Dyeing or bleaching agent according to one of claims 1 or 2, **characterized in that** the at least one amino acid of the formula (VI) is selected from arginine, lysine, histidine, asparagine, glutamine, cysteine, methionine, tryptophan and mixtures thereof, particularly preferably mixtures from arginine and lysine.

4. Dyeing or bleaching agent according to one of claims 1 to 3, **characterized in that** the at least one amino acid of the formula (VI) is in a total amount of 0.05 to 3% by weight, preferably 0.1 to 2% by weight, particularly preferably 0.2 to 1.2% by weight, converted to the undissociated amino acid and based on the weight of the dye or bleaching agent.

5. Dyeing or bleaching agent according to one of claims 1 to 4, **characterized in that** the mixtures of arginine and lysine or at least one salt of theses amino acids, are contained in a total amount of 0.05 to 3% by weight, preferably 0.1 to 2% by weight, particularly preferably 0.2 to 1.2% by weight, converted to the undissociated amino acid and based on the weight of the dye or bleaching agent.

6. Dyeing or bleaching agent according to one of claims 1-5, **characterized in that** at least one compound of the general formula (III) is contained, 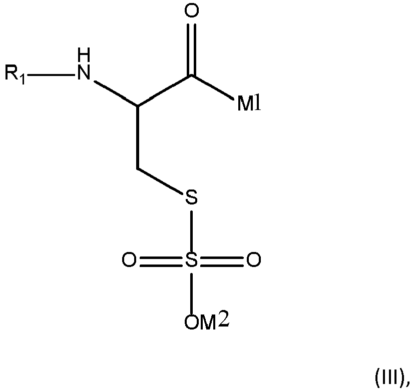 wherein
R1 is a hydrogen atom or a structural element of the formula (IV) 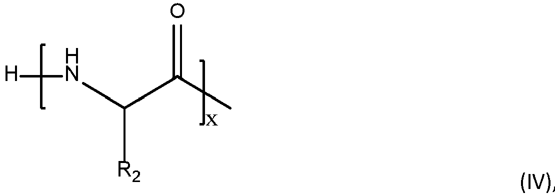 , wherein
x represents an integer from 1 to 100,
the radical R2 in each of the structural elements of the formula (IV) can be selected independently of the previous structural element of the formula (IV),
R2 represents a hydrogen atom, a methyl group, an isopropyl group, a 2-methyl propyl group, a 1-methyl-propyl group, a benzyl group, a 4-hydroxybenzyl group, a hydroxymethyl group, 1-hydroxyethyl group, a 4-aminobutyl group, a 3-carbamimidamidopropyl group, a 2-carboxyethyl group, a carboxymethyl group, a 2 -Carbamoylethyl group, a carbamoylmethyl group, a sulfanylmethyl group, a 2-(methylsulfanyl)ethyl group, a 1H-imidazol-4-ylmethyl group, a 1H-indol-3-ylmethyl group or a (sulfosulfanyl )methyl group, M1 stands for the group -OM2 or for a structural element of the formula (V) 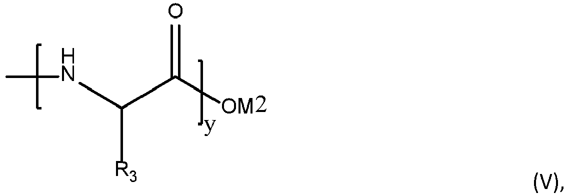 where
y stands for an integer from 1 to 100,
the radical R3 can be selected in each of the structural elements of the formula (V) independently of the previous structural element of the formula (V),
R3 represents a hydrogen atom, a methyl group, an isopropyl group, a 2-methyl-propyl group, a 1-methyl-propyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl -group, a 1H-imidazol-4-ylmethyl group, a 1H-indol-3-ylmethyl group or a (sulfosulfanyl)methyl group,
M2 represents a hydrogen atom, an equivalent of a monovalent or polyvalent cation or an ammonium ion ( NH4)+,
preferably one or more compounds of the formula (III) listed above in a total amount of 0.001 to 2.5% by weight, more preferably 0.01 to 1.0% by weight and particularly preferably 0, 02 to 0.1% by weight are contained, each based on the weight of the dyeing or bleaching agent according to the invention.

7. Dyeing or bleaching agent according to one of claims 1-6, **characterized in that** it contains 20-85% by weight, preferably 30-80% by weight of water, in each case based on the weight of the agent.

8. Dyeing or bleaching agent according to one of claims 1-7, **characterized in that** hydrogen peroxide is contained as the peroxide compound.

9. Dyeing or bleaching agent according to one of claims 1-8, **characterized in that** it contains from 0.5 to 13% by weight, preferably from 1 to 7% by weight, more preferably from 2 to 6% by weight, and particularly preferably from 3 to 4.5% by weight of hydrogen peroxide (calculated as 100% H₂O₂), in each case based on the total weight of the dyeing or bleaching agent according to the invention.

10. Dyeing or bleaching agent according to one of claims 1-9, **characterized in that** at least one polymer A is contained which has at least ten constitutive units of the formula (I), 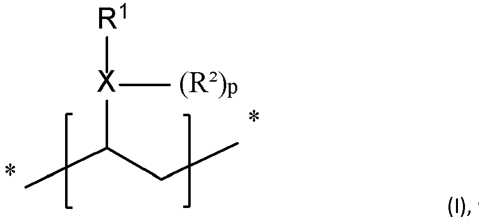 where
- X stands for nitrogen or oxygen and
- R1 and R2 each independently represent hydrogen or a C2-C10 acyl group or R1 and R2 together with is substituted with at least one C1-C6 alkyl group and/or with at least one functional group, and
- p = 0 when X represents oxygen and p = 1 when X represents nitrogen,
the polymer A not having any permanent contains ionic constitutive units, the at least one polymer A having at least ten constitutive units of the formula (I) being preferably included in a total amount of 0.2 to 5% by weight, particularly preferably 0.5 to 3% by weight, extremely preferred 1.0 to 2.3% by weight, based on the weight of the dyeing or bleaching agent.

11. Dyeing or bleaching agent according to claim 10, **characterized in that** the at least one polymer A with at least ten constitutive units of the formula (I) is selected from polymers which have 98-100 mol% constitutive units of the formula (la) 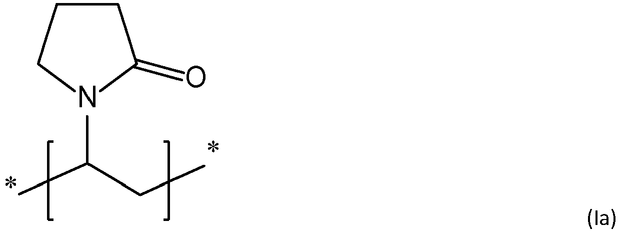 and have a degree of polymerization in the range from 40 to 1000, preferably 100 to 800, particularly preferably 350 to 650, the polymer A containing no permanent ionic constitutive units.

12. Process for the oxidative dyeing and/or lightening of keratin fibers, in particular human hair, in which a dyeing or bleaching agent according to one of claims 1-11 is applied to the keratin fibers, in particular to human hair, and after an exposure time of 0.1 to 60 Minutes, preferably 1 to 45 minutes, particularly preferably 10 to 30 minutes, is rinsed out again.

13. Process for the oxidative dyeing and/or lightening of keratin fibers, in particular human hair, which comprises the following process steps:
I. Providing a composition (A) containing
a) at least one dicarboxylic acid with 2 to 10 carbon atoms, selected from maleic acid and fumaric acid, and/or at least one salt of these acid(s),
b) at least one amino acid of the formula (VI) 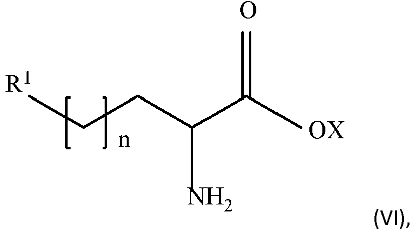 where
X represents a hydrogen atom or a monovalent or divalent cation;
n represents zero, 1, 2 or 3;
R1 represents a residue which is selected from an amino group, a guanidine group, a (1H-imidazol-4-yl) group, a carboxamide group -CONH2, a 1H-indol-3-yl group, a thiol group -SH and a methylsulfanyl group -SCH3, or at least one salt of this amino acid,
c) water and
d) optionally further at least one substance selected from - compounds of the general formula (III), 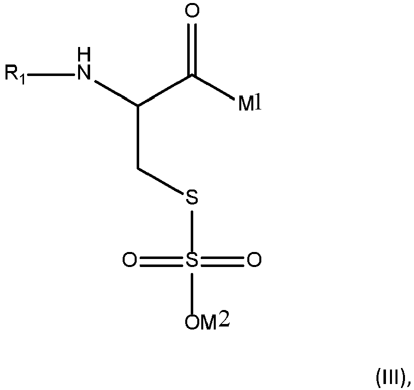 wherein
R1 is a hydrogen atom or a structural element of the formula (IV) 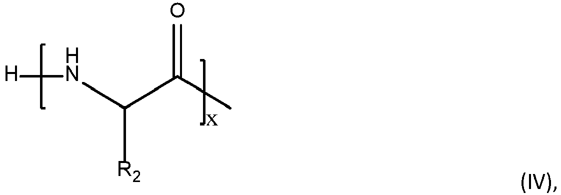 wherein
x stands for an integer from 1 to 100,
the radical R2 in each of the structural elements of the formula (IV) can be chosen independently of the previous structural element of the formula (IV),
R2 represents a hydrogen atom, a methyl group, an isopropyl group, a 2-methylpropyl group, a 1-methylpropyl group, a benzyl group, a 4-hydroxybenzyl group, a hydroxymethyl group, 1-hydroxyethyl group, a 4-aminobutyl group, a 3-carbamimidamidopropyl group , a 2-carboxyethyl group, a carboxymethyl group, a 2-carbamoylethyl group, a carbamoylmethyl group, a sulfanylmethyl group, a 2-(methylsulfanyl)ethyl group, a 1H-imidazol-4-ylmethyl group, a 1H-indol-3-ylmethyl group or a (sulfosulfanyl)methyl group, M1 represents the group -OM2 or a structural element of the formula (V) 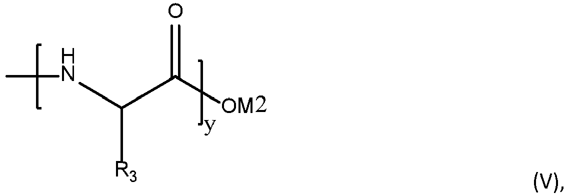 where
y represents an integer from 1 to 100,
the rest R3 in each of the structural elements of the formula (V) can be chosen independently of the previous structural element of the formula (V), R3 represents a hydrogen atom, a methyl group, an isopropyl group, a 2-methylpropyl group, a 1-methyl-propyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1H-imidazol-4-ylmethyl group, a 1H-indol-3-ylmethyl group or a (sulfosulfanyl)methyl group, M2 represents a hydrogen atom, an equivalent of a monovalent or polyvalent cation or an ammonium ion (NH4)+, and
- polymers A, which have at least ten constitutive units of the formula (I), 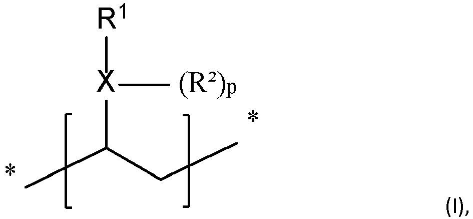 where
- X represents nitrogen or oxygen and
- R1 and R2 are each independent each other represent hydrogen or a C2-C10 acyl group or R1 and R2 together with at least one C1-C6 alkyl group and/or is substituted with at least one functional group, and
- p = 0 when X represents oxygen and p = 1 when X represents nitrogen,
the polymer A not being permanently ionic contains constitutive units,
the composition (A) preferably having a pH in the range from 3.5 to 7.1, preferably 4.5-6.5, particularly preferably 5.0 to 6.0, in each case measured at 20 ° C,
II. providing a composition (B) containing
e) at least one alkalizing agent selected from ammonium hydroxide, monoethanolamine and sodium silicates and mixtures thereof,
f) optionally water and
g) optionally at least one oxidation dye precursor and/or at least one substantive dye, the composition (B) preferably having a pH in the range from 6.5 to 11.0, preferably 8-10.5, particularly preferably 8, 5 to 10.0, each measured at 20 ° C,
III. providing a composition (C) containing
h) at least one peroxide compound, which is preferably hydrogen peroxide,
wherein the composition (C) preferably has a pH in the range from 2.5 to 6.5, preferably 3.0-5.5, particularly preferably 3.5 to 5.0, each measured at 20 ° C,
IV. mixing the compositions (A), (B) and (C) with each other, immediately afterwards
V. applying the mixture from (A), (B) and (C) on the keratin fibers, especially on human hair, and
VI. rinse out after an exposure time of 0.1 to 60 minutes, preferably 1 to 45 minutes, particularly preferably 10 to 30 minutes,
VII. optionally further hair treatments such as shaping, conditioning and / or drying.

14. Composition (A) containing
- at least one dicarboxylic acid with 2 to 10 carbon atoms selected from maleic acid and fumaric acid, in one total amount of 2 to 20% by weight, preferably 5 to 15% by weight, particularly preferably 8 to 12% by weight, in each case converted to the undissociated dicarboxylic acid and based on the weight of the composition (A), wherein the total amount of malic acid, D-tartaric acid, L-tartaric acid, meso-tartaric acid, grape acid, alpha-ketoglutaric acid, beta-ketoglutaric acid, oxaloacetic acid, maleic acid and fumaric acid is 2 to 20% by weight, preferably 5 to 15% by weight, particularly preferably 8 to 12% by weight, in each case converted to undissociated dicarboxylic acid and based on the weight of the dyeing or bleaching agent, furthermore
- at least one amino acid of the formula (VI) and / or a salt thereof in a total amount of 0.4 to 7.0% by weight, preferably 0.8 to 5.0% by weight, particularly preferably 1.5 to 4.0% by weight, in each case converted to the undissociated amino acid and based on the weight the composition (A), preferably at least one of the amino acids arginine, histidine or lysine and/or a salt thereof in a total amount of 0.4 to 7.0% by weight, preferably 0.8 to 5.0% by weight. %, particularly preferably 1.5 to 4.0% by weight, in each case converted to the undissociated amino acid and based on the weight of the composition (A), and
- water, preferably in an amount of 50-92% by weight. %, particularly preferably 60-87% by weight and extremely preferably from 65 to 80% by weight, in each case based on the weight of the composition (A),
- optionally further at least one polymer A which contains at least ten constitutive units of the formula (I), 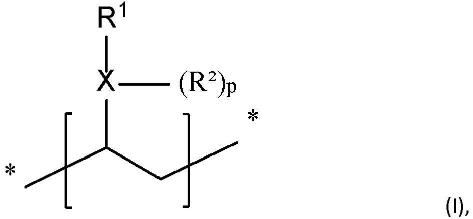 where
- X represents nitrogen or oxygen and
- R1 and R2 each independently represent hydrogen or a C2-C10 acyl group or R1 and R2 together with which optionally contains further heteroatoms, which are preferably selected from N and O, and/or is optionally substituted with at least one C1-C6 alkyl group and/or with at least one functional group, and
- p = 0 when X represents oxygen and p = 1 when X represents nitrogen,
the polymer A not having any permanent contains ionic constitutive units,
the at least one polymer A being preferably being included in a total amount of 0.5 to 14% by weight, preferably 1.0 to 11% by weight, particularly preferred 2.0 to 10% by weight, based on the weight of the composition (A),
the composition (A) preferably having a pH in the range from 3.5 to 7.1, preferably 4.5-6.5, particularly preferably 5.0 to 6.0, each measured at 20 ° C.

## Revendications

1. Agent oxydant de coloration ou de décoloration pour fibres kératiniques, en particulier pour cheveux humains, contenant
a) au moins un acide dicarboxylique ayant de 2 à 10 atomes de carbone, choisi parmi l'acide maléique et l'acide fumarique, et/ou au moins un sel de ce ou ces acides,
b) au moins un aminoacide de formule (VI) 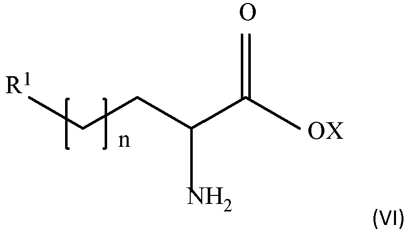 dans laquelle
X représente un atome d'hydrogène ou un cation mono- ou divalent ;
n est égal à zéro, 1, 2 ou 3 ;
R1 représente un radical choisi parmi un groupe amino, un groupe guanidine, un groupe (1H-imidazol-4-yl), un groupe amide d'acide carboxylique -CONH2, un groupe 1H-indol-3-yl, un groupe thiol -SH et un groupe méthylsulfanyl -SCH3, ou au moins un sel dudit acide aminé,
c) en outre, au moins un agent alcalinisant choisi parmi l'hydroxyde d'ammonium, la monoéthanolamine et les silicates de sodium, ainsi que leurs mélanges,
d) le cas échéant, au moins un précurseur de colorant d'oxydation et/ou au moins un colorant direct,
e) de l'eau et
f) au moins un composé peroxydé.

2. Agent de coloration ou de décoloration selon la revendication 1, **caractérisé en ce qu'**il contient au moins un acide dicarboxylique saturé ayant de 2 à 10 atomes de carbone en une quantité totale de 0,2 à 4 % en poids, de préférence de 0,33 à 3 % en poids, de manière particulièrement préférée de 0,5 à 2 % en poids, dans chaque cas converti en acide dicarboxylique non dissocié et rapporté au poids de l'agent de coloration ou de décoloration, la quantité totale d'acide malique, d'acide D-tartrique, d'acide L-tartrique, d'acide méso-tartrique, d'acide de raisin, d'acide alpha-céto-glutarique, d'acide béta-céto-glutarique, d'acide oxaloacétique, d'acide maléique et d'acide fumarique étant de 0,2 à 4 % en poids. %, de préférence de 0,33 à 3 % en poids, de manière particulièrement préférée de 0,5 à 2 % en poids, respectivement convertis en acide dicarboxylique non dissocié et rapportés au poids de l'agent de coloration ou de décoloration.

3. Agent de coloration ou de décoloration selon l'une quelconque des revendications 1 ou 2, **caractérisé en ce que** ledit au moins un acide aminé de formule (VI) est choisi parmi l'arginine, la lysine, l'histidine, l'aspa-ra-gine, la glutamine, la cystéine, la méthionine, le tryptophane et leurs mélanges, de préférence les mélanges d'arginine et de lysine.

4. Agent de coloration ou de décoloration selon l'une des revendications 1 à 3, **caractérisé en ce que** le ou les acides aminés de formule (VI) sont contenus en une quantité totale de 0,05 à 3 % en poids, de préférence de 0,1 à 2 % en poids, de manière particulièrement préférée de 0,2 à 1,2 % en poids, dans chaque cas convertis en acide aminé non dissocié et par rapport au poids de l'agent de coloration ou de décoloration.

5. Agent de coloration ou de décoloration selon l'une des revendications 1 à 4, **caractérisé en ce que** des mélanges d'arginine et de lysine ou d'au moins un sel de ces acides aminés sont contenus dans une quantité totale de 0,05 à 3 % en poids, de préférence de 0,1 à 2 % en poids, de manière particulièrement préférée de 0,2 à 1,2 % en poids, respectivement convertis en acide aminé non dissocié et rapportés au poids de l'agent de coloration ou de décoloration.

6. Agent de coloration ou de décoloration selon l'une des revendications 1 à 5, **caractérisé en ce qu'**il contient au moins un composé de formule générale (III), 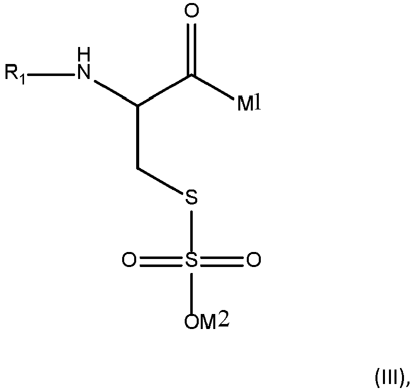 dans laquelle
R1 représente un atome d'hydrogène ou un élément structural de formule (IV) 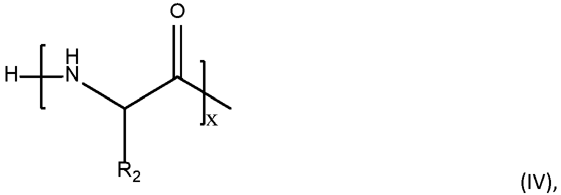 où
x représente un nombre entier de 1 à 100,
le radical R2 dans chacun des éléments structuraux de formule (IV) peut être choisi indépendamment de l'élément structural précédent de formule (IV),
R2 représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimide-amidopropyle, un groupe 2-carboxyéthyle, un groupe carboxyméthyle, un groupe 2-carboxyamoyl-éthyle, un groupe carbamoylméthyle, un groupe sulfanylméthyle, un groupe 2-(méthylsulfanyl)-éthyle, un groupe 1H-imidazol-4-ylméthyle, un groupe 1H-indol-3-ylméthyle ou un groupe (sulfosulfanyl)méthyle,
M1 représente le groupement -OM2 ou un élément structural de formule (V) 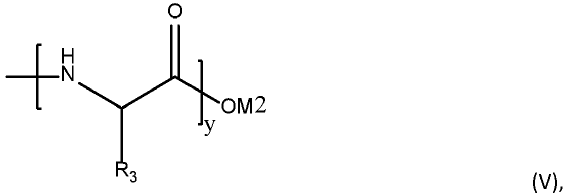 où
y représente un nombre entier de 1 à 100,
le radical R3 dans chacun des éléments structuraux de formule (V) peut être choisi indépendamment de l'élément structural précédent de formule (V),
R3 représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimide-amidopropyle, un groupe 2-carboxyéthyle, un groupe carboxyméthyle, un groupe 2-carboxyamoyl-éthyle, un groupe carbamoylméthyle, un groupe sulfanylméthyle, un groupe 2-(méthylsulfanyl)-éthyle, un groupe 1H-imidazol-4-ylméthyle, un groupe 1H-indol-3-ylméthyle ou un groupe (sulfosulfanyl)méthyle,
M2 représente un atome d'hydrogène, un équivalent d'un cation monovalent ou polyvalent ou un ion ammonium (NH4)+,
de préférence un ou plusieurs composés de formule (III) mentionnée ci-dessus étant contenus en une quantité totale de 0,001 à 2,5 % en poids, de préférence encore de 0,01 à 1,0 % en poids et de préférence encore de 0,02 à 0,1 % en poids, dans chaque cas par rapport au poids de la composition de teinture ou de décoloration selon l'invention.

7. Agent de coloration ou de décoloration selon l'une des revendications 1 à 6, **caractérisé en ce qu'**il contient de 20 à 85 % en poids, de préférence de 30 à 80 % en poids, d'eau, dans chaque cas par rapport au poids total de l'agent de coloration ou de décoloration selon l'invention.

8. Agent de coloration ou de décoloration selon l'une des revendications à 7, **caractérisé en ce qu'**il contient du peroxyde d'hydrogène comme composé peroxydé.

9. Agent de coloration ou de décoloration selon l'une des revendications 1 à 8, **caractérisé en ce qu'**il contient 0,5 à 13 % en poids, de préférence encore 1 à 7 % en poids, de préférence encore 2 à 6 % en poids et de préférence encore 3 à 4,5 % en poids de peroxyde d'hydrogène (calculé comme H2O2 à 100 %), dans chaque cas par rapport au poids total de l'agent de coloration ou de décoloration selon l'invention.

10. Agent de coloration ou de décoloration selon l'une des revendications à 9, **caractérisé en ce qu'**il contient au moins un polymère A qui présente au moins dix unités constitutives de formule (I), 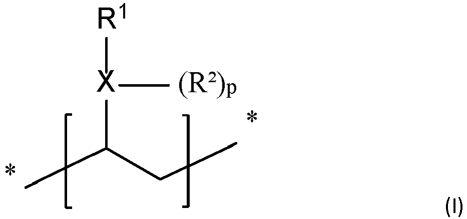 dans laquelle
X représente l'azote ou l'oxygène et
R1 et R2 représentent chacun indépendamment l'hydrogène ou un groupe acyle en C2-C10, ou R1 et R2 forment ensemble avec X un cycle saturé ou insaturé à cinq ou six chaînons, qui contient éventuellement d'autres hétéroatomes choisis de préférence parmi N et O, et/ou est éventuellement substitué par au moins un groupe alkyle en C1-C6 et/ou par au moins un groupe fonctionnel, et
p = 0 lorsque X représente l'oxygène et p = 1 lorsque X représente l'azote,
le polymère A ne contenant pas d'unités constitutives ioniques permanentes,
le au moins un polymère A avec au moins dix unités constitutives de formule (I) étant contenu de préférence dans une quantité totale de 0,2 à 5 % en poids, de manière particulièrement préférée de 0,5 à 3 % en poids, de manière exceptionnellement préférée de 1,0 à 2,3 % en poids, à chaque fois par rapport au poids de l'agent de coloration ou de décoloration.

11. Agent de coloration ou de décoloration selon la revendication 10, **caractérisé en ce que** le au moins un polymère A comportant au moins dix unités constitutives de formule (I) est choisi parmi les polymères qui comportent de 98 à 100 % en moles d'unités constitutives de formule (la) 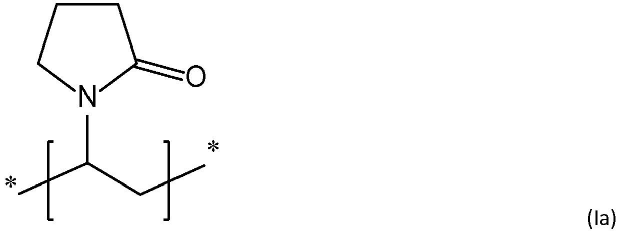 et ont un degré de polymérisation dans la plage de 40 à 1000, de préférence de 100 à 800, de manière particulièrement préférée de 350 à 650, le polymère A ne contenant pas d'unités constitutives ioniques permanentes.

12. Procédé de coloration et/ou d'éclaircissement par oxydation des fibres kératiniques, en particulier des cheveux humains, dans lequel un agent de coloration ou de décoloration selon l'une des revendications 1 à 11 est appliqué sur les fibres kératiniques, en particulier sur les cheveux humains, et est rincé après un temps d'action de 0,1 à 60 minutes, de préférence de 1 à 45 minutes, de manière particulièrement préférée de 10 à 30 minutes.

13. Procédé de coloration et/ou d'éclaircissement par oxydation des fibres kératiniques, en particulier des cheveux humains, qui comprend les étapes de procédé suivantes :
I. Préparation d'une composition (A) contenant
a) au moins un acide dicarboxylique ayant de 2 à 10 atomes de carbone, choisi parmi l'acide maléique et l'acide fumarique, et/ou au moins un sel de ce(s) acide(s),
b) au moins un aminoacide de formule (VI) 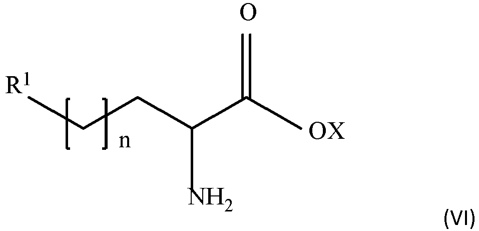 dans laquelle
X représente un atome d'hydrogène ou un cation mono- ou divalent ;
n est égal à zéro, 1, 2 ou 3 ;
R1 représente un radical choisi parmi un groupe amino, un groupe guanidine, un groupe (1H-imidazol-4-yl), un groupe amide d'acide carboxylique -CONH2, un groupe 1H-indol-3-yl, un groupe thiol -SH et un groupe méthylsulfanyl -SCH3, ou au moins un sel dudit acide aminé,
c) de l'eau, et
d) éventuellement, en outre, au moins une substance choisie parmi
- Composés de formule générale (III), 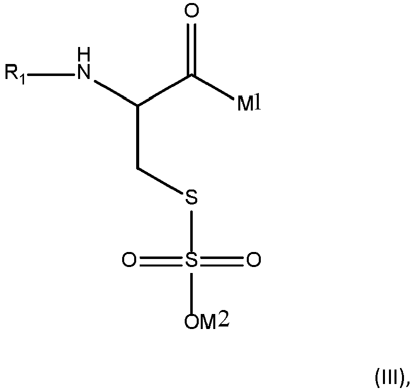 dans laquelle
R1 représente un atome d'hydrogène ou un élément structural de formule (IV) 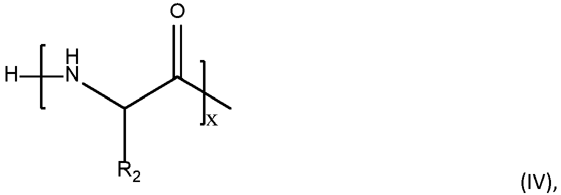
dans laquelle
x représente un nombre entier de 1 à 100,
le radical R2 dans chacun des éléments structuraux de formule (IV) peut être choisi indépendamment de l'élément structural précédent de formule (IV),
R2 représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimide-amidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)-éthyle, un groupe 1H-imidazol-4-ylméthyle, un groupe 1H-indol-3-ylméthyle ou un groupe (sulfosulfanyl)-méthyle,
M1 représente le groupement -OM2 ou un élément structural de formule (V) 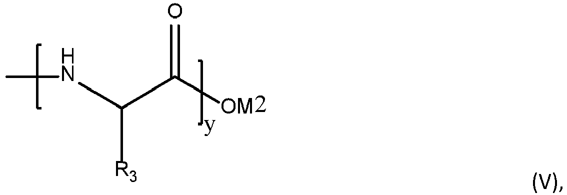 où
y représente un nombre entier de 1 à 100,
le radical R3 dans chacun des éléments structuraux de formule (V) peut être choisi indépendamment de l'élément structural précédent de formule (V),
R3 représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-amino-butyle, un groupe 3-carbamimide-amidopropyle, un groupe 2-carboxyéthyle, un groupe carboxyméthyle, un groupe 2-carboxyamoyl-éthyle, un groupe carbamoylméthyle, un groupe sulfanylméthyle, un groupe 2-(méthylsulfanyl)-éthyle, un groupe 1H-imidazol-4-ylméthyle, un groupe 1H-indol-3-ylméthyle ou un groupe (sulfosulfanyl)-méthyle,
M2 représente un atome d'hydrogène, un équivalent d'un cation monovalent ou polyvalent ou un ion ammonium (NH4)+, et
- des polymères A comportant au moins dix motifs constitutifs de formule (I), 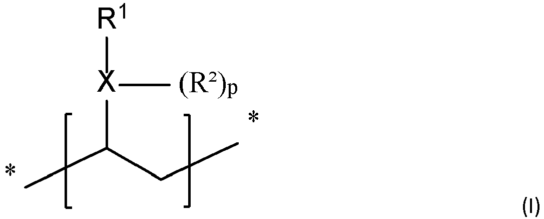 dans laquelle
X représente l'azote ou l'oxygène et
R1 et R2 représentent chacun, indépendamment l'un de l'autre, un atome d'hydrogène ou un groupe acyle en C2-C10, ou R1 et R2 forment ensemble avec X un cycle à cinq ou six chaînons, saturé ou insaturé, qui contient éventuellement d'autres hétéroatomes, choisis de préférence parmi N et O, et/ou qui est éventuellement substitué par au moins un groupe alkyle en C1-C6 et/ou par au moins un groupe fonctionnel, et
p = 0 lorsque X représente l'oxygène et p = 1 lorsque X représente l'azote,
le polymère A ne contenant pas de motifs constitutifs ioniques permanents,
la composition (A) présentant de préférence un pH dans la plage de 3,5 à 7,1, de préférence de 4,5 à 6,5, de manière particulièrement préférée de 5,0 à 6,0, dans chaque cas mesuré à 20°C,
II. préparation d'une composition (B) contenant
e) au moins un agent d'alcalinisation choisi parmi l'hydroxyde d'ammonium, la monoéthanolamine et les silicates de sodium, ainsi que leurs mélanges,
f) le cas échéant, de l'eau et
g) le cas échéant, au moins un précurseur de colorant d'oxydation et/ou au moins un colorant direct,
III. préparation d'une composition (C) contenant
h) au moins un composé peroxydé, qui est de préférence le peroxyde d'hydrogène,
IV. Mélange des compositions (A), (B) et (C) entre elles, immédiatement après.
V. Application du mélange de (A), (B) et (C) sur les fibres kératiniques, en particulier sur les cheveux humains, et V.
VI. rinçage après un temps d'action de 0,1 à 60 minutes, de préférence de 1 à 45 minutes, de préférence encore de 10 à 30 minutes,
VII. le cas échéant, d'autres traitements des cheveux, tels que la mise en forme, le conditionnement et/ou le séchage.

14. Composition (A) contenant
au moins un acide dicarboxylique ayant de 2 à 10 atomes de carbone, choisi parmi l'acide maléique et l'acide fumarique, en une quantité totale de 2 à 20 % en poids, de préférence de 5 à 15 % en poids, de manière particulièrement préférée de 8 à 12 % en poids. %, dans chaque cas convertis en acide dicarboxylique non dissocié et rapportés au poids de la composition (A), la quantité totale d'acide malique, d'acide D-tartrique, d'acide L-tartrique, d'acide méso-tartrique, d'acide tartrique racémique, d'acide alpha-céto-glutarique, d'acide béta-céto-glutarique, d'acide oxalo-acétique, d'acide maléique et d'acide fumarique étant de 2 à 20 % en poids, de préférence de 5 à 15 % en poids, de manière particulièrement préférée de 8 à 12 % en poids, respectivement convertis en acide dicarboxylique non dissocié et rapportés au poids de la composition (A), en outre
au moins un acide aminé de formule (VI) et/ou un sel de celui-ci en une quantité totale de 0,4 à 7,0% en poids, de préférence de 0,8 à 5,0% en poids, de manière particulièrement préférée de 1,5 à 4,0% en poids. %, à chaque fois converti en acide aminé non dissocié et rapporté au poids de la composition (A), de préférence au moins l'un des acides aminés arginine, his-ti-dine ou lysine et/ou un sel de ceux-ci étant présent en une quantité totale de 0,4 à 7,0 % en poids. %, de préférence de 0,8 à 5,0 % en poids, de manière particulièrement préférée de 1,5 à 4,0 % en poids, dans chaque cas calculé par rapport à l'acide aminé non dissocié et par rapport au poids de la composition (A), et
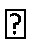 de l'eau, de préférence en une quantité de 50 à 92 % en poids, de manière particulièrement préférée de 60 à 87 % en poids et de manière extrêmement préférée de 65 à 80 % en poids, dans chaque cas par rapport au poids de la composition (A),
éventuellement, au moins un polymère A comprenant au moins dix unités constitutives de formule 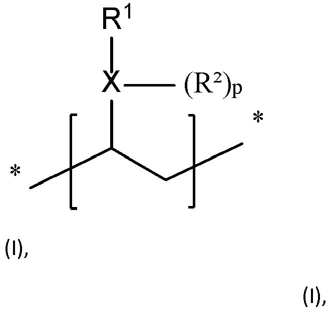 dans laquelle
X représente l'azote ou l'oxygène et
R1 et R2 représentent chacun indépendamment l'hydrogène ou un groupe acyle en C2-C10, ou R1 et R2 forment ensemble avec X un cycle saturé ou insaturé à cinq ou six chaînons, contenant éventuellement d'autres hétéroatomes choisis de préférence parmi N et O, et/ou éventuellement substitué par au moins un groupe alkyle en C1-C6 et/ou par au moins un groupe fonctionnel, et
p = 0 lorsque X représente l'oxygène et p = 1 lorsque X représente l'azote,
le polymère A ne contenant pas d'unités constitutives ioniques permanentes,
de préférence, le au moins un polymère A est contenu en une quantité totale de 0,5 à 14 % en poids, de préférence de 1,0 à 11 % en poids, de manière particulièrement préférée de 2,0 à 10 % en poids, dans chaque cas par rapport au poids de la composition (A),
la composition (A) présentant de préférence un pH dans la plage de 3,5 à 7,1, de préférence de 4,5 à 6,5, de manière particulièrement préférée de 5,0 à 6,0, mesuré dans chaque cas à 20°C.
